# EUROPÄISCHE PATENTSCHRIFT

(11) **EP 4 135 582 B1**
(45) Veröffentlichungstag und Bekanntmachung des Hinweises auf die Patenterteilung: **10.01.2024**
(21) Anmeldenummer: 21719121.2
(22) Anmeldetag: 14.04.2021
(51) Int. Cl.: A61B 7/04

(54) **ZWISCHENGLIEDER-SET FÜR EIN STETHOSKOP ZUM EINFÜGEN VOR DEN OHROLIVEN UND ZUGEHÖRIGES STETHOSKOP-SYSTEM**
INTERMEDIATE-MEMBER SET FOR A STETHOSCOPE, FOR INSERTION UPSTREAM OF THE EARPIECES, AND ASSOCIATED STETHOSCOPE SYSTEM
ENSEMBLE D'ÉLÉMENTS INTERMÉDIAIRES POUR STÉTHOSCOPE, DESTINÉ À ÊTRE INSÉRÉ EN AMONT DES ÉCOUTEURS, ET SYSTÈME DE STÉTHOSCOPE ASSOCIÉ

(30) Priorität: 15.04.2020 DE 102020204762
(43) Veröffentlichungstag der Anmeldung: 22.02.2023
(73) Patentinhaber: Kirchner & Wilhelm GmbH + Co. KG, 71679 Asperg (DE)
(72) Erfinder: KIRCHNER-GOTTSCHALK, Regina, 71706 Markgröningen (DE)
(74) Vertreter: Kohler Schmid Möbus Patentanwälte
(86) Internationale Anmeldenummer: PCT/EP2021/059694
(87) Internationale Veröffentlichungsnummer: WO 2021/209516

(56) Entgegenhaltungen:
- CN-U- 204 562 205
- CN-U- 210 811 146
- DE-A1-102018 205 672
- DE-T2- 69 637 032
- US-A- 4 048 444

## Beschreibung

Die Erfindung betrifft ein Zwischenglieder-Set für ein Stethoskop, umfassend zwei Zwischenglieder jeweils zur Anordnung zwischen einem Ohrbügelende und einer Ohrolive des Stethoskops, wobei die Zwischenglieder jeweils aufweisen
- einen Eingangskanal für eingehenden Schall und einen Eingangsanschluss des Eingangskanals zur Verbindung mit dem Ohrbügelende, und
- einen Ausgangskanal für ausgehenden Schall und einen Ausgangsanschluss des Ausgangskanals zur Verbindung mit der Ohrolive,
- eine Mikrofon-Anordnung zur Registrierung des eingehenden Schalls aus dem Eingangskanal,
- eine Verstärker-Anordnung zur elektronischen Verstärkung des eingehenden Schalls, und
- eine Lautsprecher-Anordnung zur Ausgabe des ausgehenden Schalls in den Ausgangskanal.

Ein solches Zwischenglieder-Set ist bekannt geworden aus der DE 10 2018 205 672 A1.

Stethoskope werden in vielfältiger Weise eingesetzt, um Geräusche aus dem Inneren von Patienten, etwa aus der Lunge, dem Darm oder dem Herzen, für einen Untersuchenden, etwa einen Arzt oder eine Krankenschwester, gut wahrnehmbar zu machen. Ein herkömmliches Stethoskop umfasst im Allgemeinen ein Bruststück, das auf den Patienten aufgelegt wird, eine Schlauchleitung zu zwei Ohrbügeln, die über eine Feder miteinander verbunden sind, und Ohroliven auf den Ohrbügeln, wobei im Gebrauch die Ohroliven am Gehörgang des Untersuchenden aufsitzen.

Um bei schwachen Geräuschen aus dem Inneren des Patienten diese Geräusche besser hörbar zu machen, ist es bekannt geworden, eine elektronische Verstärkung einzusetzen, beispielsweise in einem zwischen dem Bruststück und der Schlauchleitung angeordneten Zwischenglied, vgl. die DE 10 2018 205 672 A1.

Bei dem Zwischenglied aus der DE 10 2018 205 672 A1 ist vorgesehen, zwischen einem Eingangsanschluss und einem Ausgangsanschluss des Zwischenglieds einen gerade verlaufenden Verbindungskanal einzurichten, der mit einer Verschlusseinrichtung, etwa einer Irisblende, wahlweise geschlossen oder geöffnet werden kann, um in einem elektronischen Betrieb eine verbesserte akustische Trennung zwischen dem Eingangsanschluss und dem Ausgangsanschluss einzurichten, und in einem akustischen Betrieb eine Durchleitung des eingehenden Schalls ohne Verstärkung zu ermöglichen. Eine Mikrofon-Anordnung vor der Verschlusseinrichtung und eine Lautsprecheranordnung hinter der Verschlusseinrichtung sind seitlich neben dem Verbindungskanal angeordnet und über schräg verlaufende Richtkanäle an den Verbindungskanal angebunden.

Die DE 10 2018 205 672 A1 schlägt zudem in einer Variante vor, zwei solche Zwischenglieder zwischen den Ohrbügelenden und den Ohroliven einzusetzen. Eine ähnliche Anordnung ist auch aus der US 4,048,444 A bekannt geworden, wobei ebenfalls ein Mikrofon und ein Lautsprecher vor und hinter einer Verschlussblende seitlich an einem geraden Durchgangskanal angeordnet sind und senkrecht zu diesem ausgerichtet sind.

Nachteilig an diesen bekannten Bauformen ist, dass diese Zwischenglieder in axialer Richtung (entlang des Verbindungskanals) vergleichsweise viel Bauraum benötigen, sowohl für die Verschlusseinrichtung, als auch und vor allem für die seitlich angeordneten Mikrofone und Lautsprecher; letztere benötigen, um einen ausreichenden Frequenzgang bereitstellen zu können, eine gewisse Mindestgrö-ße, insbesondere Breite. Ein mit solchen axial langen Zwischengliedern an den Ohrbügelenden ausgerüstetes Stethoskop kann vom Untersuchenden als sperrig in der Handhabung empfunden werden. Wenn ein vorhandenes Stethoskop mit diesen Zwischengliedern nachgerüstet wird, muss die Feder, die die Spannung bzw. den Abstand zwischen den Ohrbügelenden vorgibt, in erheblichem Umfang nachgebogen werden. Umgekehrt, wenn das Stethoskop wieder ohne die Zwischenglieder verwendet werden soll, muss die Feder wieder in erheblichem Umfang zurückgebogen werden. Ein wiederholtes und starkes Verbiegen der Feder kann diese beschädigen.

### Aufgabe der Erfindung

Es ist Aufgabe der Erfindung, ein Zwischenglieder-Set zum Einfügen zwischen die Ohrbügelenden und die Ohroliven eines Stethoskops vorzuschlagen, mit denen ein nachgerüstetes Stethoskop weniger sperrig ist, und mit dem bei einem Wechsel zwischen einer Verwendung eines Stethoskops mit und ohne das Zwischenglieder-Set weniger oder kein Biegen an der Feder erforderlich ist.

### Beschreibung der Erfindung

Diese Aufgabe wird erfindungsgemäß gelöst durch ein Zwischenglieder-Set der eingangs genannten Art, das dadurch gekennzeichnet ist,
dass die Zwischenglieder jeweils so ausgebildet sind, dass
- der Eingangskanal oder eine eingangsseitige Schallkammer, in die der Eingangskanal mündet, und der Ausgangskanal oder eine ausgangsseitige Schallkammer, in die der Ausgangskanal mündet, durch eine feste Zwischenwand des Zwischenglieds akustisch voneinander getrennt sind,
- dass die Mikrofon-Anordnung axial endseitig am Eingangskanal und zumindest teilweise innerhalb eines radialen Erstreckungsbereichs des Eingangskanals angeordnet ist,
- und dass die Lautsprecher-Anordnung axial endseitig am Ausgangskanal und zumindest teilweise innerhalb eines radialen Erstreckungsbereichs des Ausgangskanals angeordnet ist.

Die Erfindung schlägt vor, die Mikrofon-Anordnung und die Lautsprecher-Anordnung nicht seitlich des Eingangskanals und Ausgangskanals anzuordnen, sondern endseitig und (zumindest teilweise) im radialen Erstreckungsbereich des Eingangskanals und des Ausgangskanals. Typischerweise übergreifen (bezogen auf die Fläche, und typischerweise auch bezogen auf jede radiale Richtung) die Mikrofon-Anordnung und die Lautsprecher-Anordnung wenigstens 80%, bevorzugt 100%, des radialen Erstreckungsbereichs von Eingangskanal und Ausgangskanal. Typischerweise sind die Mikrofon-Anordnung und die Lausprecher-Anordnung radial mittig zum Eingangskanal und Ausgangskanal angeordnet.

Dadurch ist es möglich, für die Mikrofon-Anordnung und die Lautsprecher-Anordnung Bauraum quer zur axialen Erstreckungsrichtung von Eingangskanal und Ausgangskanal zu nutzen, insbesondere im Bereich eines Teils des Querschnitts oder (bevorzugt) des ganzen Querschnitts des Eingangskanals und Ausgangskanals, und falls gewünscht auch radial darüber hinaus. Damit können auch vergleichsweise breite Mikrophone und Lautsprecher genutzt werden, ohne das Zwischenglied in axialer Richtung (entlang der Erstreckungsrichtung des Eingangskanals und des Ausgangskanals) größer bauen zu müssen. Damit ist bei guter akustischer Qualität, insbesondere auch bei niedrigen Frequenzen (im Bereich 15-50 Hz), ein axial kurzer Bau möglich.

Zudem sieht die Erfindung vor, den Eingangskanal und den Ausgangskanal bzw. eine eingangsseitige Schallkammer und eine ausgangsseitige Schallkammer permanent mit einer fest eingebauten (nicht öffen- und verschließbaren) Zwischenwand akustisch zu separieren. Die feste Zwischenwand ermöglicht eine bessere akustische Isolation von Eingangskanal bzw. eingangsseitiger Schallkammer und Ausgangskanal bzw. ausgangsseitiger Schallkammer als Konstruktionen mit einer beweglichen Verschlusseinrichtung. Die feste Zwischenwand ist weiterhin unmittelbar platzsparend, da eine bewegliche Verschlusseinrichtung und ein zugehöriger Betätigungsmechanismus entfallen. Zudem ist es nicht mehr nötig, den radialen Erstreckungsbereich eines Verbindungskanals bzw. des Eingangskanals und des Ausgangskanals frei zu halten, um unverstärkten Schall (zumindest zeitweise) hindurchleiten zu können. Vielmehr ist es im Rahmen der Erfindung möglich, den Eingangskanal mit der Mikrofon-Anordnung axial abzuschließen, und den Ausgangskanal mit der Lautsprecher-Anordnung axial zu beginnen, wodurch die Einkopplung der Mikrofon-Anordnung und der Lautsprecher-Anordnung gegenüber seitlichen Anordnungen deutlich verbessert wird. Dass (eingehender und ausgehender) Schall durch die Mikrofon-Anordnung und die Lautsprecher-Anordnung im jeweiligen Kanal (axial) blockiert wird, ist aufgrund des stets elektronischen Verstärkerbetriebs der Zwischenglieder unbeachtlich und sogar vorteilhaft, um die akustische Trennung von Eingang und Ausgang zu verbessern und Rücckopplungen zu vermeiden oder zu minimieren. Damit wird ebenfalls bei guter akustischer Qualität ein axial kurzer Bau ermöglicht.

Im Rahmen der Erfindung kann die axiale Baulänge der Zwischenglieder je nach Bauform im Wesentlichen auf die axiale Länge des Eingangsanschluss und des Ausgangsanschluss reduziert werden, insbesondere wenn der Eingangskanal und der Ausgangskanal quer zueinander versetzt angeordnet werden. Die Zwischenglieder sind deutlich weniger sperrig als bekannte Zwischenglieder zur Montage zwischen den Ohrbügelenden und den Ohroliven eines Stethoskops. Bei einem Wechsel zwischen einer Stethoskopverwendung mit und ohne Zwischengliedern braucht eine Feder, mit der die Spannung und der Abstand der Ohroliven bei Benutzung des Stethoskops bei einem Untersuchenden eingestellt wird, nur wenig oder meistens gar nicht nachgebogen zu werden. Dies ist für den Untersuchenden komfortabel, und minimiert die Gefahr von Beschädigungen an der Feder.

Das erfindungsgemäße Zwischenglieder-Set kann an einem herkömmlichen Stethoskop auf einfache Weise nachgerüstet werden, indem die Ohroliven von dem oder den Ohrbügeln abgenommen werden, und die Zwischenglieder zwischen den Ohrbügelenden und den Ohroliven eingesetzt werden; ggf. kann auch für jedes Zwischenglied eine zusätzliche, am Ausgangsanschuss passende Ohrolive vorgesehen sein, und beispielsweise vormontiert werden. Der Eingangsanschluss kann über verschiedene, wahlweise zu verwendende bzw. austauschbare Eingangsadapter für verschiedene Typen von Stethoskopen bzw. Ohrbügeln angepasst werden. Wenn die Energiespeicher der Zwischenglieder erschöpft sein sollten, aber dennoch das Stethoskop eingesetzt werden soll, oder wenn ein direktes (unverstärktes) Abhören von Patientengeräuschen aus anderen Gründen gewünscht ist, können die Zwischenglieder einfach ausgebaut und später wieder eingebaut werden.

Die Verstärker-Anordnung der Zwischenglieder kann insbesondere einen Mikrofon-Vorverstärker, einen Filter zur Korrektur/Umschaltung des Frequenzgangs und einen Lautsprecher-Verstärker umfassen. Einzelne Komponenten der Verstärker-Anordnung können in analoger oder in digitaler Schaltungstechnik ausgeführt sein. Mehrere Komponenten der Verstärker-Anordnung können in einem integrierten Schaltkreis zusammengefasst sein. Elektronik-Baugruppen (etwa ein Funkmodul, eine Ladeelektronik oder eine Einschaltelektronik) können Statussignale an den Lautsprecher-Verstärker senden (Signalton oder Sprachnachricht), falls gewünscht. Die Elektronik-Baugruppen können alle oder zum Teil über einen Bus kommunizieren.

### Bevorzugte Ausführungsformen des Zwischenglieder-Sets

Bei einer besonders bevorzugten Ausführungsform des erfindungsgemäßen Sets sind die Zwischenglieder jeweils so ausgebildet sind, dass
- der Ausgangskanal quer zu einer axialen Erstreckungsrichtung des Eingangskanals gegenüber dem Eingangskanal versetzt angeordnet ist. Dadurch lassen sich die Zwischenglieder in axialer Richtung besonders kompakt ausführen, und zudem lässt sich ein für einen komfortablen und abdichtenden Sitz der Ohroliven im Gehörgang des Untersuchenden ein günstiges Kippmoment erzeugen. Dafür ist beim Tragen des Stethoskops typischerweise der Eingangskanal oben und der Ausgangskanal unten angeordnet.

Eine bevorzugte Weiterbildung dieser Ausführungsform sieht vor, dass der Eingangskanal und der Ausgangskanal zumindest näherungsweise parallel zueinander ausgerichtet sind. Dadurch kann ein kompakter Aufbau weiter befördert werden, und das gewünschte Kippmoment ist einfach einzurichten. Zudem sind dann die Ohroliven, bei Nachrüstung eines herkömmlichen Stethoskops, für einen guten Sitz im Gehörgang günstig ausgerichtet.

Besonders bevorzugt ist eine Weiterbildung, bei der die Zwischenglieder jeweils so ausgebildet sind, dass
- die Mikrofon-Anordnung und die Lautsprecher-Anordnung bezüglich der axialen Erstreckungsrichtung des Eingangskanals überlappen und/oder die Lautsprecher-Anordnung bezüglich der axialen Erstreckungsrichtung des Eingangskanals näher an einer Eingangsseite des Eingangskanals liegt als die Mikrofon-Anordnung. Dadurch ist in axialer Richtung bzw. bezüglich der axialen Erstreckungsrichtung des Eingangskanals ein besonders kurzer Aufbau der Zwischenglieder möglich.

Eine besonders bevorzugte Ausführungsform sieht vor, dass die Mikrofon-Anordnung eine bevorzugte Registrierungsrichtung für Schall aufweist, die entlang der axialen Erstreckungsrichtung des Eingangskanals in den Eingangskanal gerichtet ist,
und dass die Lautsprecher-Anordnung eine bevorzugte Ausgaberichtung für Schall aufweist, die entlang der axialen Erstreckungsrichtung des Ausgangskanals in den Ausgangskanal gerichtet ist. Durch eine bevorzugte Registrierungsrichtung der Mikrofon-Anordnung und deren Ausrichtung entlang des Eingangskanals kann der Einfluss von Umgebungsgeräuschen minimiert werden. Ebenso kann durch die bevorzugte Ausgaberichtung der Lautsprecher-Anordnung und deren Ausrichtung ein Eintrag von ausgegebenem Schall in das Zwischenglied und dadurch eine Verfälschung des ausgegebenen Schalls vermindert werden. Insgesamt kann eine hohe akustische Qualität der elektronischen Verstärkung erreicht werden. Typischerweise sind die Mikrofon-Anordnung und die Lautsprecher-Anordnung auf Platinen angeordnet, die senkrecht zur axialen Erstreckungsrichtung des Eingangskanals bzw. Ausgangskanals angeordnet sind. Typischerweise sind die Mikrofon-Anordnung und die Lautsprecher-Anordnung breiter als tief ausgebildet, und die bevorzugte Registrierungsrichtung und die bevorzugte Ausgaberichtung liegen senkrecht zur jeweiligen Breiten-Richtung (also entlang der Tiefenrichtung).

Bevorzugt ist weiterhin eine Ausführungsform, bei der die Zwischenglieder jeweils so ausgebildet sind, dass der Eingangsanschluss durch einen Eingangsadapter ausgebildet ist, der in einem Gehäuse des Zwischenglieds verrastbar ist, insbesondere mit einer Bajonettmechanik. Mit diesem austauschbaren Eingangsadapter können die Zwischenglieder an unterschiedliche Stethoskope (bzw. Größen und Kopplungsmechanismen der Ohrbügelenden) auf einfache Weise angepasst werden. Im Gehäuse können Führungshilfen, insbesondere Führungsrippen, meist ausgebildet als Längsrippen, für den Eingangsadapter bei dessen Montage ausgebildet sein.

Bevorzugt ist eines Ausführungsform, bei der die Verstärker-Anordnung und ein Energiespeicher für die Verstärkeranordnung zumindest teilweise, bevorzugt überwiegend, besonders bevorzugt vollständig, in einem Bauraum angeordnet sind, der bezüglich der axialen Erstreckungsrichtung des Eingangskanals mit dem Eingangskanal überlappt. Dadurch kann Bauraum in axialer Richtung eingespart werden. Da der Eingangsanschluss in der Regel eine Hülse oder Aufnahme für das Ohrbügelende ausbildet, muss sich das Zwischenglied ohnehin in Überlappung mit dem Eingangsanschluss, der den Eingangskanal mit ausbildet, erstrecken; zudem ist der Bauraum der Überlappung mit dem Eingangskanal in der Regel weit genug vom Ohr des Untersuchenden entfernt, so dass dieser nicht gestört wird.

Vorteilhaft ist eine Ausführungsform, bei der die beiden Zwischenglieder mit Zusatz-Mikrofonanordnungen ausgestattet sind, die den Umgebungsschall aufnehmen und phasenverkehrt in die Verstärker-Anordnung einkoppeln. Mit anderen Worten, das Signal der Zusatz-Mikrofonanordnung (also der Umgebungsschall) wird vom zu verstärkenden Signal der Mikrofon-Anordnung subtrahiert. Dadurch können Umgebungsgeräusche zumindest teilweise kompensiert werden.

Bei einer vorteilhaften Ausführungsform ist vorgesehen, dass die beiden Zwischenglieder mit Funkmodulen ausgestattet sind, mittels denen die Zwischenglieder eine oder mehrere Einstellungen der Verstärker-Anordnungen, insbesondere Lautstärke-Einstellungen und/oder Frequenzgang-Einstellungen, kommunizieren können, und dass zur Vorgabe dieser einen oder mehreren Einstellungen der Verstärker-Anordnungen lediglich an einem der Zwischenglieder ein oder mehrere manuelle Bedienelemente vorgesehen sind. Dadurch ist die Benutzung des Zwischenglieder-Sets besonders komfortabel. Einstellungen der Verstärker-Anordnungen brauchen nur an einem Zwischenglied vorgenommen (vorgegeben) werden, und können dann mit beiden Zwischengliedern genutzt werden. An nur einem Zwischenglied werden manuelle Bedienelemente für die Einstellungen der Verstärker-Anordnungen benötigt, was kostengünstig ist und den Aufbau vereinfacht. Das Zwischenglied ohne die besagten Bedienelemente ("Slave") kann jedoch mit einem manuellen Ein/Aus-Schalter versehen sein, so dass nur bei eingeschaltetem Ein/Aus-Schalter das Slave-Zwischenglied betriebsbereit ist und ggf. Einstellungs-Informationen empfängt; dies spart Energie. Alternativ kann das Slave-Zwischenglied durch das Zwischenglied mit dem oder den Bedienelementen ("Master") aus der Ferne über die Funkmodule ein- und ausgeschaltet werden; in diesem Fall benötigt das Slave-Bedienelement gar keine manuellen Bedienelemente, auch keinen Ein-/Aus-Schalter. Bevorzugt können über ein Funkmodul die gemessenen Signale (des eingehenden Schalls) drahtlos an eine externe Auswertungs- und Speichereinheit geschickt werden (Smartphone, Tablet, PC).

Vorteilhaft ist weiterhin eine Ausführungsform, bei der die Zwischenglieder wiederaufladbare Energiespeicher aufweisen, insbesondere wobei die Zwischenglieder Ladeelektroniken mit Überladungs- und Tiefentladungsschutz aufweisen. Dadurch können Wechsel von Einweg-Batterien vermieden werden, was komfortabel und umweltschonend ist. Der wiederaufladbare Energiespeicher kann insbesondere als ein Elektrolytkondensator oder ein Lithium-Ionen-Akku ausgebildet sein. Die Ladeelektroniken schützen die Energiespeicher vor Schäden durch zu starke Aufladung oder Entladung.

Bei einer bevorzugten Weiterbildung dieser Ausführungsform sind an einer oder mehreren Außenseiten der Zwischenglieder Photovoltaik-Module angeordnet, mit denen die wiederaufladbaren Energiespeicher der Zwischenglieder aufladbar sind. Dadurch kann insbesondere in Einsatzgebieten, in denen ein Netzstrom nicht verfügbar oder sehr teuer ist, das Stethoskop-System bzw. das Zwischenglieder-Set kostengünstig betriebsbereit gehalten werden.

Vorteilhaft ist auch eine Ausführungsform, bei der die Eingangsanschlüsse jeweils mit einer Verdrehsicherung oder Verdrehhemmung für die Ohrbügelenden ausgebildet sind. Dadurch ist es möglich, eine Drehstellung der Zwischenglieder definiert gegenüber den Ohrbügelenden einzurichten und zu halten, insbesondere um ein gewünschtes Kippmoment und einen komfortablen Sitz der Ohroliven am Gehörgang des Untersuchenden zu erwirken. Die Verdrehsicherung oder Verdrehhemmung kann beispielhaft durch eine elastische Auskleidung der Innenseite des Eingangsadapters realisiert werden. Diese Auskleidung kann Längsrillen enthalten. In einer anderen bevorzugten Bauform enthält der Eingangsadapter in axialer Richtung angeordnete Klinken (auch als Klingen oder "Messerklingen" bezeichnet), die in radialer Richtung beweglich sind und durch Federkraft radial nach innen gegen das Ohrbügelende gedrückt werden. Durch eine Dichtlippe (radial umlaufende Dichtfläche) aus elastischem Material kann das Ende des Ohrbügels schalldicht an den Eingangskanal angekoppelt werden.

### Erfindungsgemäße Stethoskop-Systeme

In den Rahmen der vorliegenden Erfindung fällt auch ein Stethoskop-System, umfassend
- ein Stethoskop-Bruststück mit einem oder zwei Bruststück-Ausgängen,
- ein oder zwei Ohrbügel mit je einem Ohrbügel-Eingang und insgesamt zwei Ohrbügelenden,
- eine oder zwei Schlauchleitungen vom Bruststück-Ausgang oder von den Bruststück-Ausgängen zum Ohrbügel-Eingang oder zu den Ohrbügel-Eingängen,
- ein erfindungsgemäßes, oben beschriebenes Zwischenglieder-Set, wobei jeweils ein Ohrbügelende an einem Eingangsanschluss eines Zwischenglieds angeschlossen ist,
- und zwei Ohroliven, die jeweils an einem Ausgangsanschluss eines Zwischenglieds angeordnet sind. Mit anderen Worten, ein erfindungsgemäßes Stethoskop-System umfasst ein (herkömmliches) Stethoskop und ein erfindungsgemäßes, oben beschriebenes Zwischenglieder-Set, eingebaut zwischen den Ohrbügelenden und den Ohroliven. Ein solches Stethoskop-System ist komfortabel in der Verwendung und ermöglicht eine Verstärkung von Geräuschen aus dem Körperinneren von Patienten, um diese für den Untersuchenden, etwa einen Arzt oder eine Krankenschwester, besser wahrnehmbar zu machen. Die Zwischenglieder vor den Ohroliven können axial besonders kurz ausgebildet werden. Eine Feder des Stethoskop-Systems, mit der bei der Benutzung eine mechanische Spannung und der Abstand der Ohrbügelenden bzw. der Ohroliven vorgegeben wird, braucht nicht oder nur minimal gebogen (plastisch verformt) zu werden, wenn zwischen einer Verwendung mit und ohne das Zwischenglieder-Set gewechselt wird. Falls das Stethoskop-System mit nur einem (Gesamt-)Ohrbügel (und ohne eine plastisch verformbare Feder) ausgebildet ist, kann durch die geringe axiale Baugröße der Zwischenglieder der Unterschied in mechanischer Spannung und Abstand der Ohroliven bei einer Verwendung mit und ohne das Zwischenglieder-Set gering gehalten werden.

Bevorzugt ist eine Ausführungsform des erfindungsgemäßen Stethoskop-Systems, wobei der Ausgangskanal quer zur Erstreckungsrichtung des Eingangskanals gegenüber dem Eingangskanal versetzt angeordnet ist, bei der die Zwischenglieder so an dem Ohrbügel oder den Ohrbügeln angeordnet sind, dass die Eingangskanäle dem Ohrbügeleingang oder den Ohrbügeleingängen abgewandt sind, und die Ausgangskanäle dem Ohrbügeleingang oder den Ohrbügeleingängen zugewandt sind. Mit anderen Worten, bei normalem Gebrauch des Stethoskops befindet sich der Eingangsanschluss bei einem jeweiligen Zwischenglied oberhalb des Ausgangsanschlusses. Durch das Eigengewicht des Stethoskops entsteht dann ein Kippmoment, das die Ohrolive auf den Gehörgang drückt, wodurch der Gehörgang mit der Ohrolive gut (d.h. praktisch luftdicht und weitgehend schalldicht) verschlossen werden kann, wodurch der Untersuchende Geräusche aus dem Stethoskop besonders gut wahrnehmen kann.

Bevorzugt ist auch eine Ausführungsform des Stethoskop-Systems, wobei die Zwischenglieder jeweils einen wiederaufladbaren Energiespeicher aufweisen, weiterhin umfassend eine Ladestation mit zwei virtuellen Ohren, an denen die an dem oder den Ohrbügeln montierten Zwischenglieder mit den Ohroliven einhängbar sind, wobei im eingehängten Zustand die Zwischenglieder so angeordnet sind, dass die Ladestation die wiederaufladbaren Energiespeicher der Zwischenglieder aufladen kann. Dadurch kann das mit dem Zwischenglieder-Set ausgestattete Stethoskop einfach und griffbereit gelagert und dabei zuverlässig geladen werden. Das Aufladen kann induktiv oder über ohmsche Ladekontakte erfolgen. Die Ladestation hat typischerweise einen Netzanschluss (Anschluss für extern über ein Versorgungsnetz bereitgestellten Strom) zur Energieversorgung, beispielsweise über ein externes oder internes Netzteil, insbesondere über ein USB-Ladegerät und/oder über einen USB-Ladeport. Alternativ oder zusätzlich kann die Ladestation ein Photovoltaik-Modul zur Energieversorgung aufweisen. Sollen die Zwischenglieder ohne Stethoskop geladen werden, werden sie bevorzugt über eine Klemmvorrichtung in der Ladestation fixiert.

Besonders bevorzugt ist eine Weiterbildung dieser Ausführungsform, bei der der Abstand zwischen den beiden virtuellen Ohren variabel einstellbar ist. Dadurch wird erreicht, dass unabhängig von der Kopfweite des Untersuchenden (also des Verwenders des Stethoskops) der oder die Ohrbügel mit den Zwischengliedern bequem in die Ladestation eingehängt werden können und dort optimal aufliegen, sodass eine zuverlässige Aufladung erfolgt. Die Ladestation kann beispielsweise ein verstellbares Teleskoprohr umfassen, an dessen Enden sich die Aufnahmen für die Zwischenglieder befinden.

Bevorzugt ist eine Weiterbildung, bei der die Ladestation ausgebildet ist mit
- einem auf die Ladestation aufsteckbaren Photovoltaik-Modul zur Energieversorgung, und
- einem Kabel zur Verbindung des Photovoltaik-Moduls mit der Ladestation zumindest im abgesteckten Zustand des Photovoltaik-Moduls. Das abgesteckte und über ein Kabel verbundene Photovoltaik-Modul kann an einem Ort mit hoher Sonneneinstrahlung, z. B. an ein Fenster, angebracht werden, um die Ladeleistung zu verbessern. Gleichzeitig kann die (übrige) Ladestation an einem geschützten Ort verbleiben und insbesondere fest installiert werden, etwa an einer Gebäudewand.

Vorteilhaft ist eine Ausgestaltung dieser Weiterbildung, bei der die Ladestation einen wiederaufladbaren Puffer-Energiespeicher umfasst. Über diesen Puffer-Energiespeicher können die wiederaufladbaren Energiespeicher der Zwischenglieder auch nachts (oder allgemeiner zu einer Zeit, wenn kein ausreichendes Sonnenlicht zur Verfügung steht) aufgeladen werden. In Arbeitspausen oder bei leeren Energiespeichern des Zwischenglieds ist eine Schnellladung möglich. Der Puffer-Energiespeicher kann über das Photovoltaik-Modul über längere Zeit aufgeladen werden, während das Stethoskop benutzt wird und daher nicht an der Ladestation eingehängt ist.

Bevorzugt ist auch eine Weiterbildung, die vorsieht, dass die Ladestation mit einer Diebstahlsicherung ausgebildet ist, umfassend zwei verfahrbare Platten, die bei an den virtuellen Ohren angeordneten Zwischengliedern von außen auf Anschlag an die Zwischenglieder und/oder an den oder die Ohrbügel, an dem oder denen die Zwischenglieder montiert sind, heranfahrbar und am Anschlag verrastbar sind, wobei die Verrastung der Platten mit einem Schloss aufhebbar ist. Mit der Diebstahlsicherung kann ein Diebstahl des Stethoskop-Systems bzw. der Zwischenglieder auf komfortable Weise verhindert oder zumindest erschwert werden.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt eine schematische Darstellung eines erfindungsgemäßen Stethoskop-Systems, mit zwischen den Ohrbügelenden und den Ohroliven eingebauten Zwischengliedern;
- Fig. 2: zeigt einen schematischen Längsschnitt durch ein Zwischenglied für ein erfindungsgemäßes Zwischenglieder-Set, in einer Ausführungsform mit in Querrichtung zueinander versetztem Eingangskanal und Ausgangskanal;
- Fig. 3a: zeigt eine schematische Stirnansicht des Zwischenglieds von Fig. 2, auf der Eingangsseite;
- Fig. 3b: zeigt eine schematische Stirnansicht des Zwischenglieds von Fig. 2, auf der Ausgangsseite;
- Fig. 4: zeigt einen schematischen Längsschnitt durch eine erste Bauform eines Eingangsadapters für die Erfindung;
- Fig. 5a: zeigt einen schematischen Längsschnitt durch eine zweite Bauform eines Eingangsadapters für die Erfindung, mit radial federnden Klinken;
- Fig. 5b: zeigt einen schematischen Querschnitt durch die zweite Bauform des eines Eingangsadapters von Fig. 5a in der dortigen Ebene A-A;
- Fig. 6: zeigt eine schematische Übersicht einer ersten Bauform einer Elektronik eines Zwischenglieds für die Erfindung, mit analogem Ein/Aus-Schalter,
- Fig. 7: zeigt eine schematische Übersicht einer zweiten Bauform einer Elektronik eines Zwischenglieds für die Erfindung, mit digitalem Ein/Aus-Schalter;
- Fig. 8: zeigt eine schematische Übersicht einer dritten Bauform einer Elektronik eines Zwischenglieds für die Erfindung, ausgebildet als Master-Zwischenglied mit Funkmodul;
- Fig. 9: zeigt eine schematische Übersicht einer vierten Bauform einer Elektronik eines Zwischenglieds für die Erfindung, ausgebildet als Slave-Zwischenglied mit Funkmodul, mit manuellem digitalem Ein/Aus-Schalter;
- Fig. 10: zeigt eine schematische Übersicht einer fünften Bauform einer Elektronik eines Zwischenglieds für die Erfindung, ausgebildet als Slave-Zwischenglied mit Funkmodul, mit automatischem digitalem Ein/Aus-Schalter;
- Fig. 11: zeigt schematisch in Frontalansicht ein erfindungsgemäßes StethoskopSystem eingehängt an einer beispielhaften Ladestation, für die Erfindung;
- Fig. 12: zeigt einen schematischen Längsschnitt durch die Ladestation von Fig. 11 auf der linken Seite;
- Fig. 13: zeigt eine schematische Stirnansicht auf die Ladestation von Fig. 11 von der linken Seite;
- Fig. 14a: zeigt eine Variante der Ladestation von Fig. 11, mit einer Diebstahlsicherung, in schematischer Stirnansicht von der linken Seite;
- Fig. 14b: zeigt die Variante der Ladestation von Fig. 14a, in einer schematischen Ansicht von unten;
- Fig. 15: zeigt einen schematischen Längsschnitt durch ein Zwischenglied für ein erfindungsgemäßes Zwischenglieder-Set, in einer Ausführungsform mit koaxialem Eingangskanal und Ausgangskanal.

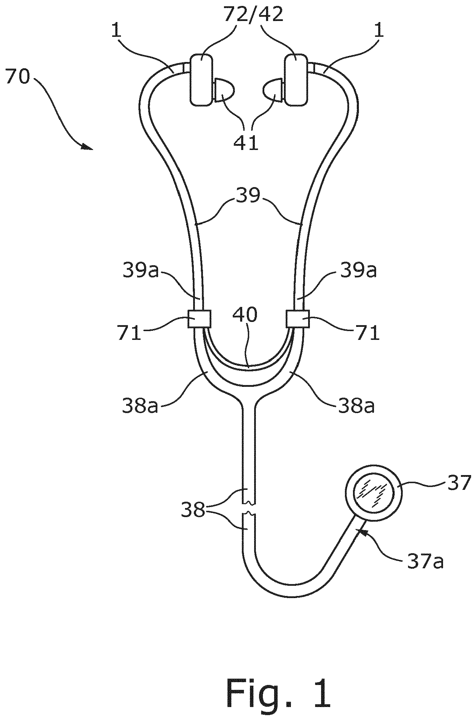

Die **Fig. 1** zeigt in einer schematischen Gesamt-Ansicht ein beispielhaftes Stethoskop-System 70 gemäß der Erfindung.

In der gezeigten Bauform umfasst das Stethoskop-System 70 ein Bruststück 37, das auf den Körper eines Patienten aufgesetzt werden kann, etwa auf dessen Brust. Das Bruststück 37 ist an einem Bruststück-Ausgang 37a an eine Schlauchleitung (Schlauchsystem) 38 angeschlossen, die sich auf zwei Schlauchenden 38a verzweigt. Die Schlauchleitung 38 ist typischerweise aus Gummi gefertigt. Jedes Schlauchende 38a ist an einem Übergangsstück 71 an einen Ohrbügel 39 an dessen Ohrbügel-Eingang 39a angeschlossen. Die Ohrbügel 39 sind typischerweise aus Metall, etwa Edelstahl, gefertigt. An den Übergangsstücken 71 ist hier auch eine Feder 40 befestigt, die die Ohrbügel 39 positioniert und deren mechanische Spannung zueinander in Abhängigkeit von der Aufbiegung der Ohrbügel 39 festlegt. Die Feder 40 besteht typischerweise aus Metall, in der Regel ebenfalls Edelsta h l.

Am Ohrbügelende 1 eines jeden Ohrbügels 39 ist ein Zwischenglied 42 angeordnet, mit dem der vom Bruststück 37 kommende Schall elektronisch registriert und verstärkt wird. Die Zwischenglieder 42 sind an einem jeweiligen Ausgangsanschluss mit Ohroliven 41 versehen, die auf den Gehörgang eines jeweiligen Ohrs eines Untersuchenden (Anwender des Stethoskop-Systems 70) aufgesetzt werden können, um aus den Zwischengliedern 42 auf den Ausgangsanschluss bzw. die Ohroliven 41 gegebenen, verstärkten Schall anzuhören.

Die beiden Zwischenglieder 42 bilden ein erfindungsgemäßes Zwischenglieder-Set 72, das insbesondere an einem vorhandenen Stethoskop nachgerüstet werden kann.

In alternativen Bauformen, die nicht näher dargestellt sind, kann das Bruststück auch mit zwei Bruststückausgängen versehen sein, an denen zwei Schlauchleitungen befestigt sind, die zu den Ohrbügeleingängen führen. Ebenso ist es möglich, einen durchgehenden Gesamt-Ohrbügel für beide Ohren vorzusehen, der mit ausreichender Elastizität versehen ist (und beispielsweise aus Kunststoff gefertigt ist) und keine zusätzliche Feder benötigt, und einen gemeinsamen Ohrbügeleingang für die Schlauchleitung vom Bruststück aufweist, ebenfalls nicht näher dargestellt.

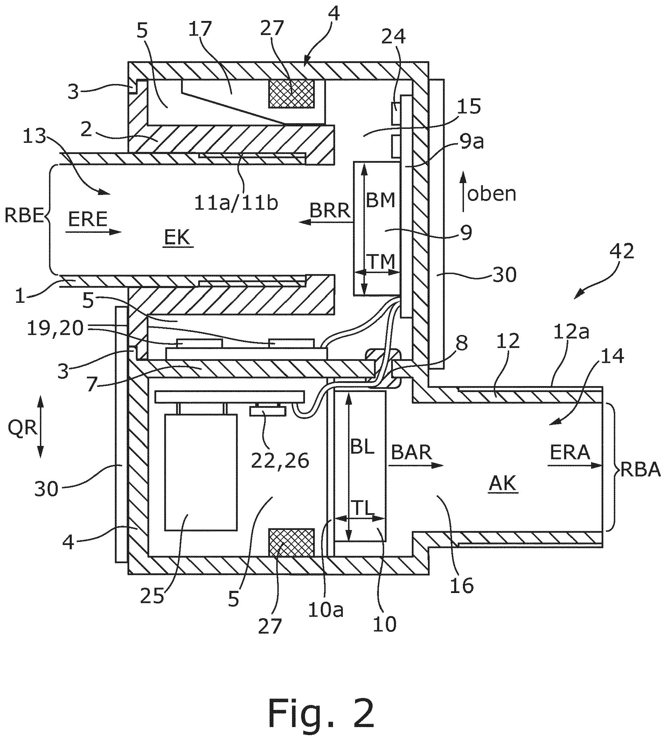

Die **Fig. 2** zeigt schematisch eine bevorzugte Ausführungsform eines Zwischenglieds 42 (auch genannt Zwischenverstärker) für die Erfindung.

Das Zwischenglied 42 bildet einen akustischen Eingangsanschluss 13 aus, an dem ein Eingangsadapter 2 in einem Gehäuse 4 des Zwischenglieds 42 befestigt ist. Zur Befestigung des Eingangsadapters 2 im Gehäuse 4 wird hier eine Bajonettmechanik 3 verwendet, die eine Festlegung einer (Rotations-)Orientierung des Eingangsadapters 2 im Gehäuse 4 gestattet. Die Einführung des Eingangsadapters 2 in das Gehäuse 4 bei der Montage wird mit Führungshilfen, die hier als Längsrippen 17 ausgebildet sind, erleichtert.

Im Eingangsadapter 2 ist das Ohrbügelende 1 befestigt, hier verschraubt. Dazu sind am Ohrbügelende 1 ein Außengewinde 11a und am Eingangsadapter 2 ein komplementäres Innengewinde 11b ausgebildet. Man beachte, dass auch andere Strukturen zur Befestigung des Ohrbügelendes 1, insbesondere zur drehfesten Befestigung und luftdichten Abdichtung des Ohrbügelendes 1 im Eingangsadapter 2 vorgesehen sein können, etwa basierend auf Steckmechanismen. Der austauschbare Eingangsadapter 2 ermöglicht eine Anpassung des Zwischenglieds 42 an verschiedene Größen und/oder Typen von Ohrbügeln bzw. Ohrbügelenden 1.

Durch das Ohrbügelende 1, das in das Zwischenglied 42 eingeschraubt ist, wird der größte Teil eines Eingangskanals EK für eingehenden Schall aus dem Bruststück ausgebildet. Der Eingangskanal EK erstreckt sich in einer gerade verlaufenden Erstreckungsrichtung ERE in das Zwischenglied 42 in eine eingangsseitige Schallkammer 15 hinein.

Am Ende des Eingangskanals EK ist eine Mikrofon-Anordnung 9 auf einer Platine 9a angeordnet, wobei die Platine 9a senkrecht zur Erstreckungsrichtung ERE des Eingangskanals EK ausgerichtet ist. Die Mikrofon-Anordnung 9 ist innerhalb des radialen Erstreckungsbereichs RBE des Eingangskanals EK angeordnet, d.h. sie greift in diesen radialen Erstreckungsbereich RBE ein und übergreift ihn hier sogar vollständig und reicht über diesen radial hinaus. Ihre bevorzugte Registrierungsrichtung BRR ist direkt axial in den Eingangskanal EK gerichtet, entlang der Erstreckungsrichtung ERE.

Die Mikrofon-Anordnung 9 besitzt eine Breite BM quer zur Erstreckungsrichtung ERE, wobei die Breite BM hier deutlich größer ist als eine axiale Tiefe TM entlang der Erstreckungsrichtung ERE. In der gezeigten Ausführungsform und auch allgemein bevorzugt gilt BM≥0,8*RBE, bevorzugt BM≥1*RBE und weiterhin BM≥1,33*TM, bevorzugt BM≥1,5*TM, besonders bevorzugt BM≥2*TM.

Die eingangsseitige Schallkammer 15, in der die Mikrofon-Anordnung 9 angeordnet ist, ist von einer ausgangsseitigen Schallkammer 16, in der eine Lautsprecher-Anordnung 10 angeordnet ist, durch eine Zwischenwand 7 dauerhaft akustisch getrennt. Die Zwischenwand 7 verfügt insbesondere über keine öffen- und schließbare Durchgänge für Schall, um diesen direkt (unverstärkt) vom Eingangskanal EK in den Ausgangskanal AK des Zwischenglieds 42 zu leiten. An einem Durchlass 8 für Kabel (auch genannt Kabeldurchlass) in der Zwischenwand 7 ist eine akustische Abdichtung vorgenommen, etwa mittels eines erstarrten Kunstharzes oder eines Silikonkautschuks.

Der Ausgangskanal AK des Zwischenglieds 42 wird hier größtenteils durch einen Montagestutzen (Außenstruktur) 12 ausgebildet, an dessen radialer Außenseite hier ein Außengewinde 12a zum Aufschrauben einer Ohrolive (letztere nicht dargestellt in Fig. 2) ausgebildet ist; man beachte, dass auch andere Befestigungsstrukturen, etwa für ein Aufstecken der Ohrolive und insbesondere umfassend in Umfangsrichtung verlaufenden Rillen (nicht näher dargestellt), möglich sind. Der Ausgangskanal AK erstreckt sich gerade entlang einer Erstreckungsrichtung ERA des Ausgangskanals AK. Durch den Montagestutzen 12 wird hier der Ausgangsanschluss 14 des Zwischenglieds 42 ausgebildet.

Die Lautsprecheranordnung 10 ist auf einer Platine 10a an einem Ende des Ausgangskanals AK angeordnet, wobei die Platine 10a senkrecht zur Erstreckungsrichtung ERA des Ausgangskanals AK ausgerichtet ist. Die Lautsprecher-Anordnung 10 ist innerhalb des radialen Erstreckungsbereichs RBA des Ausgangskanals AK angeordnet, d.h. sie greift in diesen radialen Erstreckungsbereich RBA ein und übergreift ihn hier sogar vollständig und reicht radial über diesen hinaus. Ihre bevorzugte Ausgaberichtung BAR ist direkt axial in den Ausgangskanal AK gerichtet, entlang der Erstreckungsrichtung ERA.

Die Lautsprecher-Anordnung 10 besitzt eine Breite BL quer zur Erstreckungsrichtung ERA, wobei die Breite BL hier deutlich größer ist als eine axiale Tiefe TL entlang der Erstreckungsrichtung ERA. In der gezeigten Ausführungsform und auch allgemein bevorzugt gilt BL≥0,8*RBA, bevorzugt BL≥1*RBA und weiterhin BL≥1,33*TL, bevorzugt BL≥1,5*TL, besonders bevorzugt BL≥2*TL.

In der gezeigten Ausführungsform sind der Eingangskanal EK und der Ausgangskanal in einer Querrichtung QR, die senkrecht zur Erstreckungsrichtung ERE des Eingangskanals EK verlauft, zueinander versetzt angeordnet. Der Versatz in Querrichtung ist so groß, dass der Eingangskanal EK und der Ausgangskanal AK in Querrichtung QR nicht überlappen. Eingangskanal EK und Ausgangskanal AK verlaufen hier parallel zueinander.

Die Mikrofon-Anordnung 9 und die Lautsprecher-Anordnung 10 sind so angeordnet, dass diese in axialer Richtung (entlang der Erstreckungsrichtung ERE des Eingangskanals EK) teilweise miteinander überlappen. Bezogen auf die Erstreckungsrichtung ERE des Eingangskanals EK liegt die platinennahe Seite (Rückseite) der Lautsprecher-Anordnung 10 sogar näher zur (ohrbügelseitigen) Eingangsseite des Eingangskanals EK als die platinenabgewandte Seite (Vorderseite) der Mikrofon-Anordnung 9. Die bevorzugte Registrierungsrichtung BRR der Mikrofon-Anordnung 9 und die bevorzugte Ausgaberichtung BAR der Lautsprecher-Anordnung 10 sind bezüglich der axialen Richtung zueinander entgegengesetzt.

Das Zwischenglied 42 ist mit einer Verstärker-Anordnung ausgebildet, die hier insbesondere einen Mikrofon-Vorverstärker 19, einen Filter 20 zur Korrektur oder Umschaltung des Frequenzgangs (analog oder digital) und einen Lautsprecher-Verstärker 22 umfasst. Diese Bauteile der Verstärker-Anordnung sind in der gezeigten Ausführungsform alle in einem Bauraum 5 untergebracht, der in Querrichtung QR neben dem Eingangskanal EK bzw. neben dem Eingangsanschluss 13 liegt, also in Erstreckungsrichtung ERE des Eingangskanals EK mit dem Eingangskanal EK bzw. dem Eingangsanschluss 13 überlappt. Dabei sind hier der Mikrofon-Vorverstärker 19 und der Filter 20 sowie ein optionales Funkmodul 24 (inklusive Antenne) in der eingangsseitigen Schallkammer 15, und der Lautsprecher-Verstärker 22 sowie ein Energiespeicher 25 und eine Ladeelektronik 26 in der ausgangsseitigen Schallkammer 16 untergebracht.

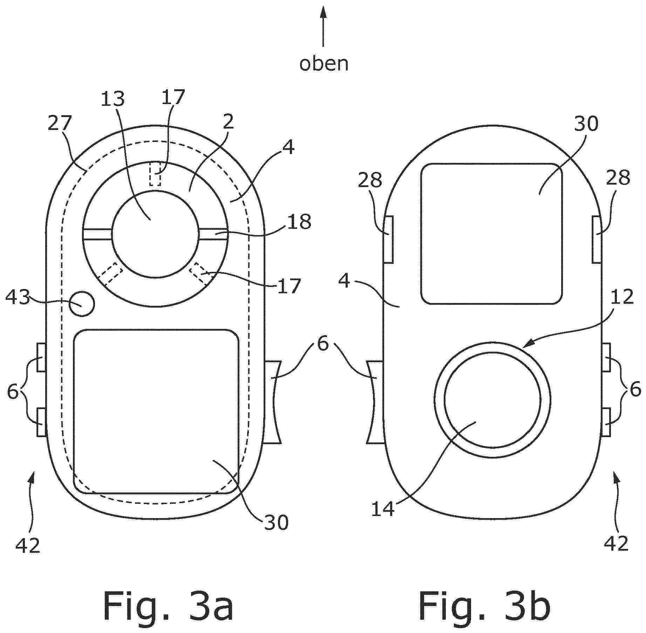

Der Energiespeicher 25 ist wiederaufladbar ausgebildet, und kann hier mittels einer Ladespule 27 für eine induktive Ladung aufgeladen werden. Alternativ oder zusätzlich kann das Zwischenglied 42 auch über Photovoltaik-Module 30 an der Außenseite des Gehäuses 4 verfügen, oder auch über ohmsche Ladekontakte verfügen (in Fig. 2 verdeckt, vgl. aber Fig. 3b hierzu).

Das Zwischenglied 42 versetzt die Ohrolive am Stethoskop, verglichen mit einem herkömmlichen Stethoskop ohne Zwischenglied vor der Ohrolive, um maximal 12 mm, bevorzugt maximal 10 mm axial nach innen.

Die **Fig. 3a** mit einer eingangsseitigen Stirnansicht und die **Fig. 3b** mit einer ausgangsseitigen Stirnansicht illustrieren das Zwischenglied 42 näher.

Das Zwischenglied 42 verfügt typischerweise über Bedien- und Anzeigeelemente 6, mit denen beispielsweise das Zwischenglied 42 eingeschaltet und ausgeschaltet werden kann, der Einschaltzustand optisch überwacht werden kann, eine Lautstärke-Einstellung verändert werden kann, oder auch eine Filter-Einstellung der Verstärker-Anordnung verändert werden kann. Typische Anzeige- und Bedienelemente umfassen Druckschalter, Wippschalter und Leuchtdioden.

An der eingangsseitigen Stirnseite (Fig. 3a) kann der Eingangsadapter 2, in welchem hier zur besseren Übersicht kein Ohrbügel montiert ist, erkannt werden. Dieser kann mit einem Schlitz 18 betätigt (verrastet und entrastet) werden, etwa indem eine Münze in den Schlitz 18 eingesteckt wird und gedreht wird, um ihn im Gehäuse 4 zu befestigen oder aus dem Gehäuse 4 zu lösen. Auch sind die Einführhilfen in Gestalt von Längsrippen 17 schematisch angedeutet (die gestrichelte Kontur deutet an, dass sie vom Eingangsadapter 2 verdeckt werden). Zudem ist ein Zusatz-Mikrofonanordnung 43 an der Außenseite des Gehäuses 4 vorgesehen, mit der Umgebungsgeräusche registriert werden. Deren registrierte Signale können phasenverkehrt bei der Verstärkung berücksichtigt und dadurch im verstärkten Schall minimiert werden.

An der ausgangsseitigen Stirnseite (Fig. 3b) sind zudem optionale ohmsche Ladekontakte 28 und die Photovoltaik-Module 30 auf der Außenseite des Gehäuses 4 erkennbar, über die ebenfalls das Zwischenglied 42 bzw. dessen Energiespeicher aufgeladen werden kann.

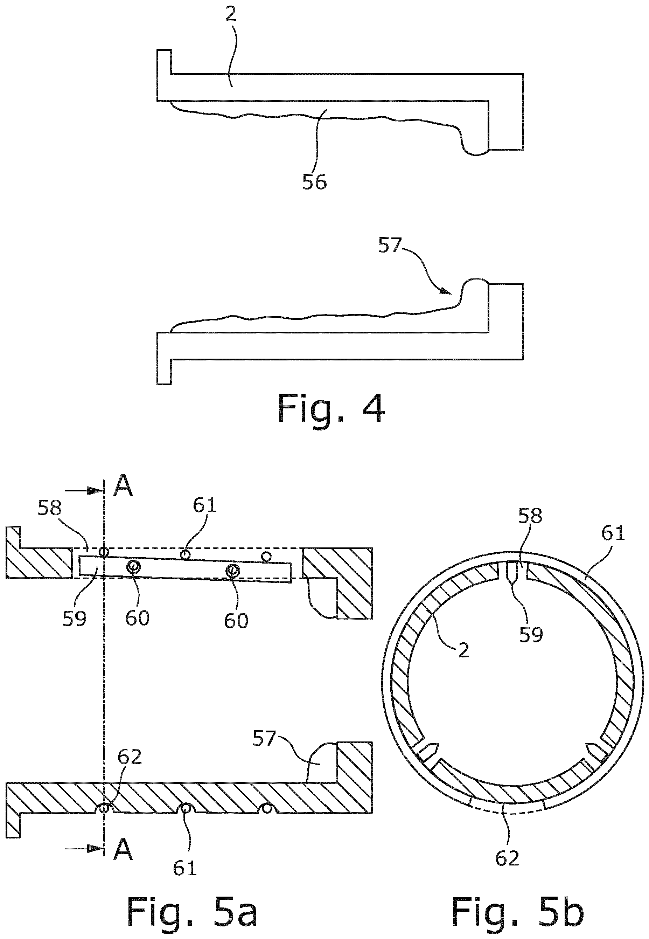

Der Eingangsadapter 2 stellt für den Ohrbügel am Ohrbügelende ein lösbares, aber verdrehungssteifes und möglichst luftdicht abschließendes Befestigungssystem bereit. Alternativ zur in Fig. 2 gezeigten Verschraubung kann, wie in **Fig. 4** dargestellt, auch ein elastischer Einsatz 56 mit einer elastischen Dichtlippe 57 im Eingangsadapter 2 vorgesehen sein. Das Ohrbügelende (nicht dargestellt) wird einfach in den Eingangsadapter 2 eingeschoben und im elastischen Einsatz 56 radial verklemmt, und liegt mit seinem vorderen Ende an der Dichtlippe 57 an.

Für eine besonders sichere Sperre eines Ohrbügelendes gegen ein Verdrehen im Eingangsadapter 2 kann der Eingangsadapter 2, wie in **Fig. 5a** im Längsschnitt und in **Fig. 5b** im Querschnitt in Ebene A-A gezeigt, einen Satz von Klinken (auch genannt Messerklingen) 59 aufweisen. Die Klinken 59 verlaufen in Längsschlitzen 58 des Eingangsadapters 2 und sind über Haltestifte 60 gehalten sowie mittels Federringen 61 nach radial innen vorgespannt, wobei axial weiter hinten (tiefer im Zwischenglied) die Kinken 59 weiter nach innen ragen als axial weiter vorne. Dadurch wird ein Ohrbügelende (nicht dargestellt) bei seiner Einführung geführt und zentriert. Die Federringe 61 sind außen am Adapter 2 in umlaufenden Nuten 62 geführt. Am Grund des Eingangsadapters 2 ist eine elastische Dichtlippe 57 vorgesehen, um einen luftdichten Abschluss für das Ohrbügelende einzurichten.

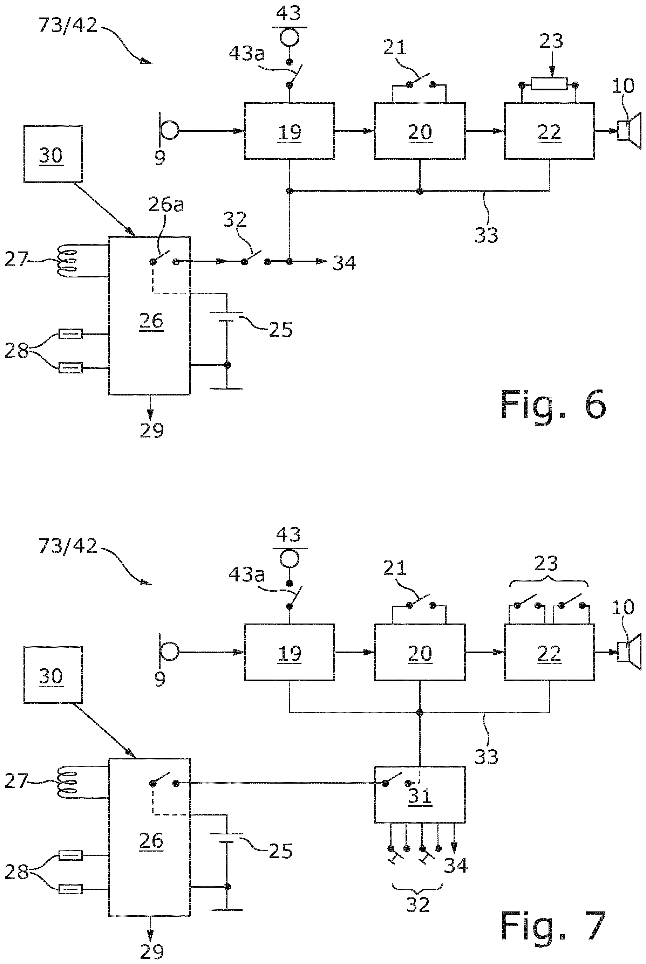

Die **Fig. 6** zeigt eine beispielhafte Verstärker-Anordnung 73 eines Zwischenglieds 42 für die Erfindung in einer ersten Bauform.

Die Verstärker-Anordnung 73 umfasst den Mikrofon-Vorverstärker 19, der an die Mikrofon-Anordnung 9 angeschlossen ist, um dessen Signal zu verstärken. Das vorverstärkte Signal wird an den Filter 20 weitergeleitet. Falls eine Zusatz-Mikrofonanordnung 43 zur Registrierung von Umgebungsgeräuschen vorhanden ist, kann deren Signal bei der Verstärkung in Abzug gebracht werden; die Zusatz-Mikrofonanordnung 43 bzw. die Umgebungsgeräuschkompensation kann über einen Schalter 43a ein- und ausgeschaltet werden.

Mit dem Filter 20 kann der Frequenzgang des eingegangenen Signals verändert werden ("Filter"). In einem einfachen Fall kann dabei eine Filterfunktion mit einem Schalter 21 ein- und ausgeschaltet werden. Es kann aber auch eine stets wirkende Filterfunktion vorgesehen sein, dann ist der Schalter 21 nicht vorhanden. Falls gewünscht können auch mehrere verschiedene Filterfunktionen über den Schalter 21 und ggf. weitere Schalter ausgewählt werden.

Das gefilterte Signal wird in den Lautsprecher-Verstärker 22 geleitet, wo dieses verstärkt wird und der Lautsprecher-Anordnung 10 zugeleitet wird. Dabei kann die Verstärkung mit einem Lautstärke-Steller 23 eingestellt werden, beispielsweise durch einen veränderlichen Widerstand oder in einfacheren Fällen durch einen Wippschalter.

Die Elektronik-Bauteile 19, 20 und 22 werden hier über einen gemeinsamen Versorgungsspannungs-Anschluss 33 mit Betriebsspannung versorgt, die vom Energiespeicher 25 bereitgestellt wird. Der Energiespeicher 25 ist über eine Ladeelektronik 26 mit Überladungs- und Tiefentladeschutz mit einem Schalter 32 verbunden, über den die gesamte Verstärkung ein- und ausgeschaltet werden kann. Bei geschlossenem (eingeschaltetem) Schalter 32 wird hier auch ein Indikator 34 (etwa eine Leuchtdiode) mit Spannung versorgt und dadurch aktiviert, mit dem der Einschaltzustand angezeigt wird. Die Ladeelektronik 26 schaltet mit einem internen Schalter 26a die Spannung am gemeinsamen Versorgungsspannungs-Anschluss 33 ab, wenn der Energiespeicher 25 einen zu niedrigen (Beschädigungen am Energiespeicher 25 verursachenden) Ladezustand zu erreichen droht. Weiterhin entkoppelt die Ladeelektronik 26 in nicht näher dargestellter Weise den Energiespeicher 25 von der oder den Quellen von Ladestrom, wenn der Energiespeicher 25 einen zu hohen (Beschädigungen am Energiespeicher 25 verursachenden) Ladezustand zu erreichen droht.

Die Ladeelektronik 26 kann Energie aus der Ladespule 27 beziehen, die typischerweise an eine Ladespule einer Ladestation (siehe unten) induktiv ankoppelbar ist. Alternativ oder zusätzlich kann eine Energieversorgung der Ladeelektronik 26 aus Photovoltaik-Modulen 30 und/oder aus ohmschen Ladekontakten 28, etwa Ladekontaktstreifen, erfolgen. Der Ladestand des Energiespeichers 25 kann zudem über einen Indikator 29 (etwa eine Leuchtdiode mit wechselnder Farbe) angezeigt werden.

Die Verstärker-Anordnung 73 kann analog oder auch ganz oder teilweise digital ausgebildet werden. In der **Fig. 7** ist eine zweite Bauform einer Verstärker-Anordnung 73 eines Zwischenglieds 42 dargestellt, wobei nur die wesentlichen Unterschiede zur ersten Bauform von Fig. 6 erläutert werden.

In der zweiten Bauform von Fig. 7 ist eine Einschaltelektronik 31 vorgesehen, die die Verbindung zwischen der Ladeelektronik 26 und dem gemeinsamen Versorgungsspannungs-Anschluss 33 herstellt. Die Einschaltelektronik 31 verfügt insbesondere über eine zeitgesteuerte automatische Abschaltung, die beispielsweise fünf Minuten nach einem Einschalten an den Schaltern 32 die Verstärker-Anordnung 73 abschaltet, wenn diese bis dahin nicht manuell an den Schaltern 32 abgeschaltet wurde. Die Schalter 32 sind hier als separate Druckschalter zum Einschalten und Ausschalten ausgebildet; alternativ kann beispielsweise auch nur ein einzelner Druckschalter 32 zum Wechseln des Einschaltzustands an der Einschaltelektronik 31 vorgesehen sein.

In der gezeigten Bauform ist zudem der Lautstärke-Steller 23 mit zwei separaten Druckschaltern zum Steigern und Absenken der digital geregelten Verstärkung in diskreten Schritten vorgesehen.

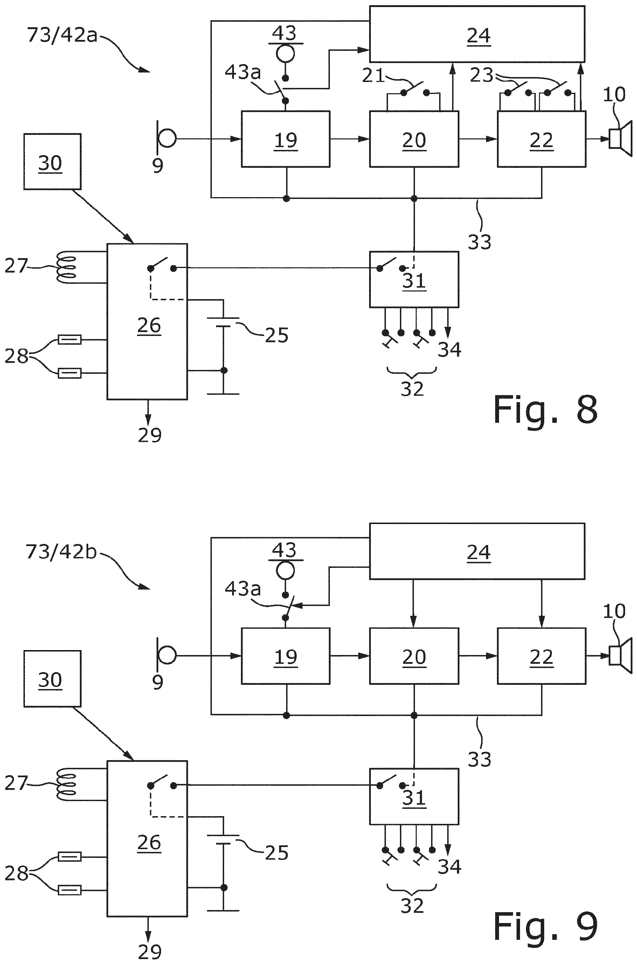

Die in **Fig. 8** gezeigte dritte Bauform einer Verstärker-Anordnung 73 ist mit einem Funkmodul 24 ausgestattet und für ein Master-Zwischenglied 42a eines Zwischenglieder-Sets vorgesehen; es werden nur die wesentlichen Unterschiede zur Bauform von Fig. 7 erläutert.

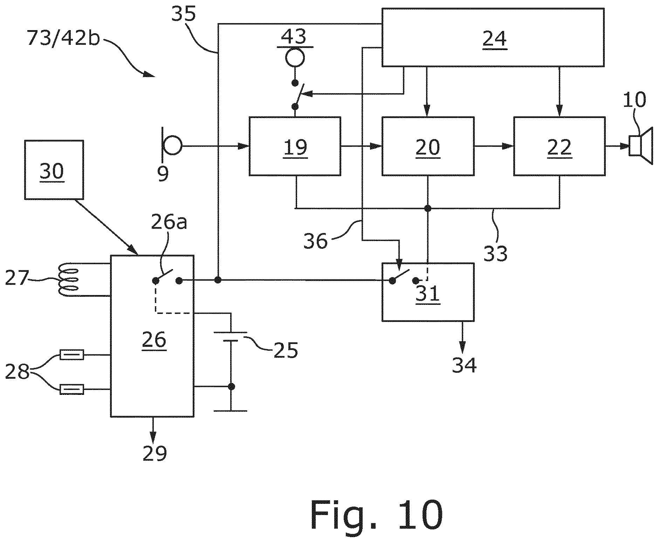

Das Funkmodul 24 liest die manuell eingestellten Verstärker-Einstellungen der Schalter 43a, 21 und 23 aus und gibt diese über Funk an ein Slave-Zwischenglied des Zwischenglieder-Sets (siehe Fig. 9 und Fig. 10) weiter. Das Funkmodul 24 kann beispielsweise Wlan, Bluetooth oder LoRa-Übertragung anwenden. Das Funkmodul 25 verfügt bevorzugt über eine integrierte Funkantenne (nicht näher dargestellt), oder ist an die Ladespule des Zwischenglieds 42a angeschlossen, die dann auch als Funkantenne benutzt wird. Das Funkmodul 24 ist dabei an den gemeinsamen Spannungsversorgungs-Anschluss 33 angebunden; dadurch ist dem Funkmodul 24 auch indirekt bekannt, ob das Master-Zwischenglied 42a mit den Schaltern 32 an der Einschaltelektronik 31 eingeschaltet wurde, und es kann diese Information über Funk ebenfalls an das Slave-Zwischenglied weitergeben.

In **Fig. 9** ist eine vierte Bauform einer Verstärker-Anordnung 73 für ein Slave-Zwischenlied 42b eines Zwischenglieder-Sets gezeigt; es werden nur die wesentlichen Unterschiede zur Bauform von Fig. 8 erläutert.

Das Funkmodul 24 empfängt über Funk vom Master-Zwischenglied gewünschte Verstärker-Einstellungen und steuert diese am Mikrofon-Vorverstärker 19 bzw. am Schalter 43a der Zusatz-Mikrofonanordnung 43 (Umgebungsgeräusche-Kompensation ein/aus), am Filter 20 (Frequenzgang-Einstellung) und am Lautsprecher-Verstärker 22 (Lautstärke-Einstellung) an. Das Funkmodul 24 wird hier über den gemeinsamen Spannungsversorgungs-Anschluss 33 mit Energie versorgt. Die Verstärker-Anordnung 73 wird über die Einschaltelektronik 31 und die die dortigen Schalter 32, manuell ein- und ausgeschaltet. Dadurch kann Energie gespart werden. Abgesehen von den Schaltern 32 sind keine manuellen Bedienelemente am Slave-Zwischenglied 42b vorgesehen; insbesondere gibt es keine manuellen Schalter zur Verstellung der Frequenzgang-Einstellung oder der Lautstärke-Einstellung.

In **Fig. 10** ist eine alternative fünfte Bauform der Verstärker-Anordnung eines Slave-Zwischenlieds 42b eines Zwischenglieder-Sets gezeigt; es werden nur die wesentlichen Unterschiede zu Fig. 9 erläutert.

Die Einschaltelektronik 31 wird in dieser Bauform über das Funkmodul 24 ein- und ausgeschaltet. Die Einschaltelektronik 31 ist dazu über eine Steuerleitung 36 mit dem Funkmodul 24 verbunden. Ob das Slave-Zwischenglied 42b eingeschaltet werden soll, wird über Funk vom Master-Zwischenglied an das Funkmodul 24 des Slave-Zwischenglieds 42b mitgeteilt. Das Funkmodul 24 ist über einen separaten Versorgungsspannungs-Anschluss 35 unabhängig von der Schaltstellung der Einschaltelektronik 31 mit dem Energiespeicher 25 verbunden, damit das Funkmodul 24 auch bei im Übrigen ausgeschalteter Verstärker-Anordnung 73 die Einschaltelektronik 31 schalten kann, insbesondere einschalten kann.

Bei dieser Bauform benötig das Slave-Zwischenglied 42b gar keine manuellen Bedienelemente, auch nicht zum Ein- und Ausschalten der Einschaltelektronik 31.

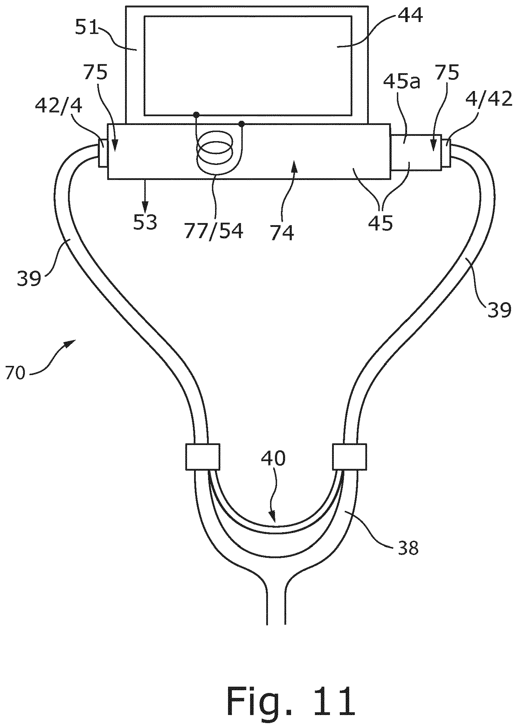

Die **Fig. 11** zeigt in einer Übersicht ein erfindungsgemäßes Stethoskop-System 70, wobei dessen Stethoskop zum Laden in einer Ladestation 74 angeordnet ist.

Die Ladestation 74 bildet zwei virtuelle Ohren 75 aus, das heißt zwei Halterungen zum Einhängen der Ohroliven bzw. der Zwischenglieder 42 des Stethoskop-Systems 74, die in einem Abstand näherungsweise entsprechend dem Abstand der zwei Ohren eines menschlichen Kopfs, in etwa entsprechend der Kopfgröße des Untersuchenden (Anwenders des Stethoskops), angeordnet sind. Zur Verstellung des Abstands der beiden virtuellen Ohren 75 ist das Lademodul 74 mit einem verstellbaren Teleskoprohr 45 ausgebildet; dessen innerer Teil 45a kann in Fig. 11 nach rechts unterschiedlich weit ausgezogen und die Verstellposition in nicht näher dargestellter Weise fixiert, etwa verklemmt, werden. Durch die Verstellbarkeit des Teleskoprohrs 45 kann eine Anpassung an die Kopfgröße des Untersuchenden bzw. an die Einstellung der Feder 40 und damit an die Weite der Ohrbügel 39 des Stethoskop-Systems 70 erfolgen.

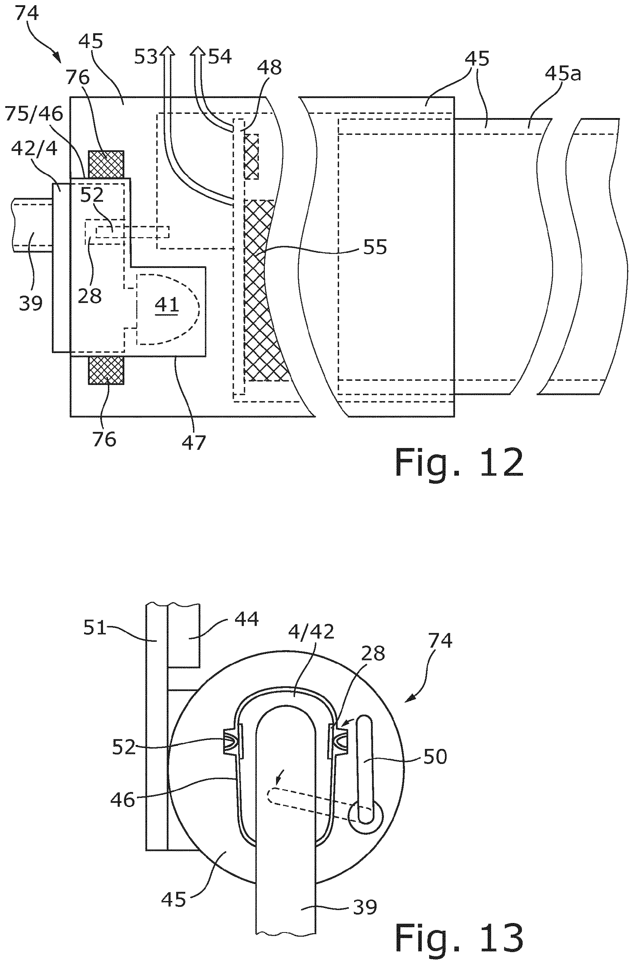

Das Lademodul 74 ist hier mit einer Wandhalterung 51 ausgebildet, an der ein aufsteckbares und abnehmbares Photovoltaik-Modul 44 angeordnet ist. Mit dem Photovoltaik-Modul 44 kann die Ladestation 74 mit Ladestrom versorgt werden. Wird das Photovoltaik-Modul 44 abgenommen und beispielsweise an einem sonnigen Fenster platziert, bleibt es über ein Kabel 77 mit der Ladestation 74 verbunden. Das Kabel 77 bildet den Anschluss 54 zur Ladestation 74 bzw. dessen Puffer-Energiespeicher aus (nicht dargestellt in Fig. 11, vgl. aber Fig. 12 hierzu). Der Puffer-Energiespeicher kann mit dem Photovoltaik-Modul 44 aufgeladen werden, auch wenn das Stethoskop-System 70 gerade benutzt wird und daher nicht an der Ladestation 74 angeordnet ist. Die im Puffer-Energiespeicher gespeicherte Energie kann dann später, wenn das Stethoskop-System 70 in der Ladestation 74 angeordnet ist, für eine Schnellladung oder eine Nachtladung eingesetzt werden.

Die Kraft der Feder 40 drückt die an den Ohrbügeln 39 angeordneten Zwischenglieder 42 auf bzw. in die virtuellen Ohren 75, wodurch die Zwischenglieder 42 für das Aufladen ihrer Energiespeicher korrekt platziert gehalten werden.

Die **Fig. 12** erläutert die Ladestation 74 im Bereich des linken Endes des Teleskoprohrs 45 von Fig. 11 in einem schematischen Längsschnitt näher; das rechte Ende (nicht dargestellt) ist in analoger Weise ausgebildet.

Zur Ausbildung des virtuellen Ohren 75 weist die Ladestation 74 eine näherungsweise ovale Aussparung 46 für das Zwischenglied 42 auf, die an ihrem Grund eine Ausnehmung 47 zur Aufnahme der Ohrolive 41 aufweist. Um die Aussparung 46 herum verläuft eine Ladespule 76 der Ladestation 74 zur induktiven Kopplung mit der Ladespule (vgl. Bezugszeichen 27 in Fig. 2) des Zwischenglieds 42, um das Zwischenglied 42 aufzuladen. Alternativ oder zusätzlich können Federkontakte 52 der Ladestation auf die Ladekontakte 28 des Zwischenglieds 42 drücken, um einen ohmschen Kontakt zum Laden des Zwischenglieds 42 herzustellen.

Auf einer Platine 48 ist der Puffer-Energiespeicher 55 angeordnet und angeschlossen, in welchem Energie, das das Photovoltaik-Modul über den Anschluss 54 bereitstellt, zwischengespeichert wird, bis eine Ladung der Zwischenglieder 42 (in einer Benutzungspause des Stethoskops, etwa in der Nacht) erfolgen kann. Alternativ oder zusätzlich zum Photovoltaik-Modul kann ein Anschluss 53 zu einem Stromnetz vorgesehen sein; die Platine 48 kann dann auch ein Netzteil tragen oder an ein externes Netzteil (z. B. USB-Ladegerät) angeschlossen sein.

In der stirnseitigen Ansicht von **Fig. 13** auf die linke Seite der Ladestation 74 von Fig. 11 und Fig. 12 kann nochmals die ovale Aussparung 46 im Teleskoprohr 45 ersehen werden, in welcher das Zwischenglied 42 angeordnet ist. Ebenso können die Federkontakte 52, die auf die Ladekontakte 28 drücken, gut ersehen werden. Das Teleskoprohr 45 ist an der Wandhalterung 51 gehalten, an der hier das Photovoltaik-Modul 44 aufgesteckt ist.

In der gezeigten Bauform ist zudem ein einschwenkbare Klemmvorrichtung 50 vorgesehen, mit der das Zwischenglied 42 in der ovalen Aussparung 46 gehalten werden kann, wenn das Zwischenglied 42 ohne den Ohrbügel 39 in der Ladestation 74 geladen werden soll. In diesem Fall hintergreift die eingeschwenkte Klemmvorrichtung 50 (anstelle des Ohrbügels 39) das Zwischenglied 42.

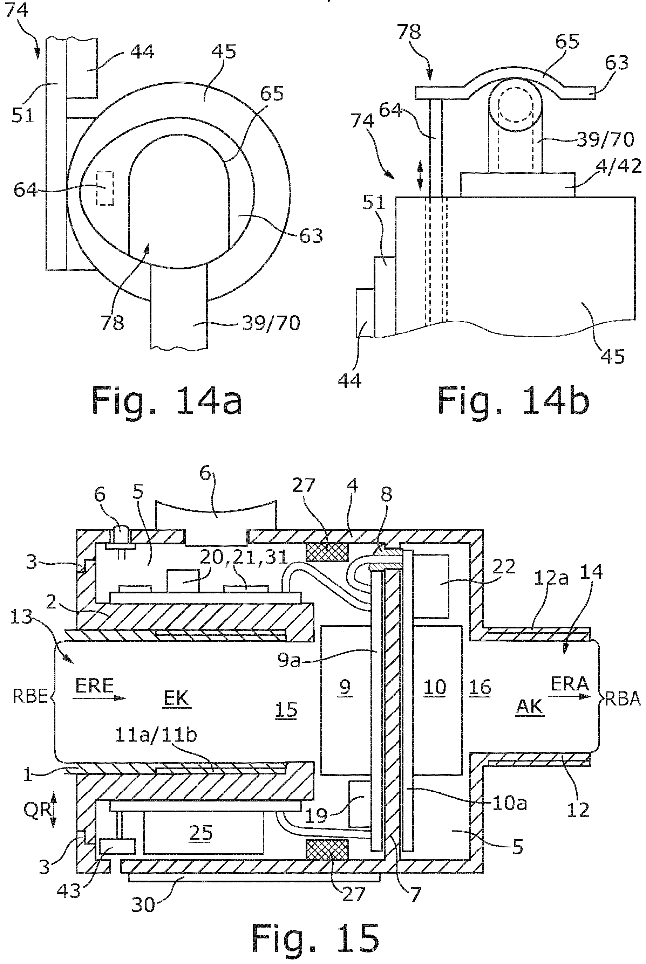

Falls gewünscht, kann wie in **Fig. 14a** in Stirnansicht und **Fig. 14b** in Ansicht von unten auch eine Diebstahlsicherung 78 an der Ladestation 74 ausgebildet sein, die typischerweise anstelle der Klemmvorrichtung 50 vorgesehen ist.

Die Diebstahlsicherung 78 umfasst in der gezeigten Bauform auf jeder Seite eine stirnseitig angeordnet Platte 63, die verdrehungssicher auf einem Haltestab 64 mit hier rechteckigen Querschnitt befestigt ist. Die Haltestäbe 64 sind jeweils innerhalb des Teleskoprohrs 45 verdrehungssicher geführt und können jeweils über einen einseitig wirkenden Rastmechanismus auf Anschlag auf die Ohrbügel 39 eines an den virtuellen Ohren eingehängten Stethoskop-Systems 70 und/oder auf die in die virtuellen Ohren eingesteckten Zwischenglieder 42 gedrückt werden. Damit ist eine Entnahme des Stethoskop-Systems 70 oder der Zwischenglieder 42 nicht mehr möglich. Zur Entsperrung werden mit Hilfe eines im Teleskoprohr 45 angebrachten Schlosses (nicht näher dargestellt) die beiden Rastmechanismen simultan entkoppelt, sodass die Platten 63 wieder abgehoben werden können.

Die Platten 63 können eine Wölbung 65 enthalten, etwa eine leichte Längswölbung wie hier dargestellt, um besser auf dem Ohrbügel 39 zu sitzen.

Die **Fig. 15** zeigt eine alternative Bauform eines Zwischenglieds 42 für die Erfindung. Diese Bauform entspricht weitgehend der in Fig. 2 gezeigten Bauform, so dass nachfolgend nur die wesentlichen Unterschiede erläutert werden.

Bei der in Fig. 15 gezeigten Bauform sind der Eingangskanal EK und der Ausgangskanal AK koaxial zueinander angeordnet, so dass die Erstreckungsrichtungen ERE und ERA von Eingangskanal EK und Ausgangskanal AK zusammenfallen, und der Eingangskanal EK und der Ausgangskanal AK folgen in axialer Richtung aufeinander. Die fest eingebaute Zwischenwand 7, die die eingangsseitige Schallkammer 15 und die ausgangsseitige Schallkammer 16 akustisch trennt, ist senkrecht zur axialen Richtung (Erstreckungsrichtung ERE des Eingangskanals EK) ausgerichtet. Die Platinen 9a, 10a sind an gegenüberliegenden Seiten der Zwischenwand 7 angeordnet.

Die Mikrofon-Anordnung 9 ist wiederum endseitig am Eingangskanal EK eingreifend in (und hier vollständig übergreifend) dessen radialen Erstreckungsbereich RBE angeordnet, und die Lautsprecher-Anordnung 10 ist wiederum endseitig am Ausgangskanal AK eingreifend in (und hier vollständig übergreifend) dessen radialen Erstreckungsbereich RBA angeordnet.

Ein Teil der Verstärker-Anordnung mit dem Mikrofon-Vorverstärker 19 und dem Filter 20 sowie weiterhin der Energiespeicher 25 sind in der eingangsseitigen Schallkammer 15 untergebracht. Ein weiterer Teil der Verstärker-Anordnung, nämlich der Lautsprecher-Verstärker 22, ist in der ausgangsseitigen Schallkammer 16 untergebracht. Der Mikrofon-Vorverstärker 19 ist auf der Platine 9a, und der Lautsprecher-Verstärker 22 ist auf der Platine 10a angeordnet. Der Filter 20, dessen Schalter 21, die Einschaltelektronik 31 und auch der Energiespeicher 25 sind hier in einem Bauraum 5 in axialer Richtung überlappend mit dem Eingangskanal EK bzw. dem Eingangsanschluss 13 angeordnet.

In der gezeigten Bauform sind der Eingangskanal EK und der Ausgangskanal AK (einschließlich der Mikrofon-Anordnung 9 und der der Lautsprecher-Anordnung 10) bzw. deren gemeinsame Achse bezüglich der Querrichtung QR näherungsweise mittig im Zwischenglied 42 bzw. in dessen Gehäuse 4 ausgebildet. Der Eingangskanal EK und der Ausgangskanal AK (einschließlich der Mikrofon-Anordnung 9 und der Lautsprecher-Anordnung 10) bzw. deren gemeinsame Achse können alternativ auch exzentrisch zur zentralen Längsachse des Gehäuses 4 (also bezüglich der Querrichtung QR außermittig) angeordnet sein, z. B. quer zu dieser Längsachse nach unten versetzt (nicht näher dargestellt). Im letzteren Fall hat der Bauraum 5 zur Aufnahme der Elektronik im oberen Teil des Gehäuses 4 einen größeren Querschnitt als im unteren Teil, wodurch größere elektronische Komponenten, insbesondere der Energiespeicher, besser untergebracht werden können.

Die gezeigte Bauform von Fig. 15 ist in axialer Richtung ebenfalls recht kompakt ausgebildet, aber nicht ganz so kompakt wie die Bauform von Fig. 2. Jedoch ist die Bauform von Fig. 15 in Querrichtung QR deutlich kompakter als die Bauform von Fig. 2 ausgebildet.

### Bezugszeichenliste

- 1: Ohrbügelende
- 2: Eingangsadapter
- 3: Bajonettmechanik
- 4: Gehäuse
- 5: Bauraum
- 6: Bedien- und Anzeigeelemente
- 7: Zwischenwand
- 8: akustisch abgedichteter Kabeldurchlass
- 9: Mikrofon-Anordnung
- 9a: Platine (für Mikrofon-Anordnung)
- 10: Lautsprecher-Anordnung
- 10a: Platine (für Lautsprecher-Anordnung)
- 11a: Außengewinde (am Ohrbügelende)
- 11b: Innengewinde (am Eingangsadapter)
- 12: Montagestutzen
- 12a: Außengewinde (Montagestutzen)
- 13: Eingangsanschluss
- 14: Ausgangsanschluss
- 15: eingangsseitige Schallkammer
- 16: ausgangsseitige Schallkammer
- 17: Längsrippe als Führungshilfe
- 18: Schlitz
- 19: Mikrofon-Vorverstärker
- 20: Filter
- 21: Schalter
- 22: Lautsprecher-Verstärker
- 23: Lautstärke-Steller
- 24: Funkmodul
- 25: Energiespeicher
- 26: Ladeelektronik mit Überladungs- und Tiefentladeschutz
- 26a: integrierter Schalter
- 27: Ladespule (Zwischenglied)
- 28: Ladekontakte
- 29: Indikator für Ladezustand
- 30: Photovoltaik-Modul (Zwischenglied)
- 31: Einschaltelektronik
- 32: Schalter
- 33: gemeinsamer Spannungsversorgungs-Anschluss
- 34: Indikator (für Einschaltzustand)
- 35: separater Versorgungsspannungs-Anschluss
- 36: Steuerleitung
- 37: Bruststück
- 37a: Bruststück-Ausgang
- 38: Schlauchleitung
- 38a: Schlauchende
- 39: Ohrbügel
- 39a: Ohrbügel-Eingang
- 40: Feder (Ohrbügel)
- 41: Ohrolive
- 42: Zwischenglied
- 42a: Master-Zwischenglied
- 42b: Slave-Zwischenglied
- 43: Zusatz-Mikrofonanordnung
- 43a: Schalter
- 44: Photovoltaik-Modul (Ladestation)
- 45: Teleskoprohr
- 45a: innerer Teil
- 46: ovale Aussparung
- 47: Ausnehmung für Ohrolive
- 48: Platine (für Puffer-Energiespeicher)
- 50: Klemmvorrichtung
- 51: Wandhalterung
- 52: Federkontakte
- 53: Anschluss zu Stromnetz
- 54: Anschluss des Photovoltaik-Moduls zur Ladestation
- 55: Puffer-Energiespeicher
- 56: elastischer Einsatz
- 57: Dichtlippe
- 58: Längsschlitz
- 59: Klinke (Messerklinge)
- 60: Haltestift
- 61: Federring
- 62: umlaufende Nut
- 63: Platte zur Diebstahlsicherung
- 64: Haltestab
- 65: Wölbung
- 70: Stethoskop-System
- 71: Übergangsstück
- 72: Zwischenglieder-Set (Set)
- 73: Verstärker-Anordnung
- 74: Ladestation
- 75: virtuelles Ohr
- 76: Ladespule (Ladestation)
- 77: Kabel
- 78: Diebstahlsicherung
- AK: Ausgangskanal
- BAR: bevorzugte Ausbreitungsrichtung
- BL: Breite Lautsprecher-Anordnung
- BM: Breite Mikrofon-Anordnung
- BRR: bevorzugte Registrierungsrichtung
- EK: Eingangskanal
- ERA: Erstreckungsrichtung des Ausgangskanals
- ERE: Erstreckungsrichtung des Eingangskanals
- QR: Querrichtung
- RBA: Radialer Erstreckungsbereich Ausgangskanal
- RBE: Radialer Erstreckungsbereich Eingangskanal
- TL: Tiefe Lautsprecher-Anordnung
- TM: Tiefe Mikrofon-Anordnung

## Patentansprüche

1. Zwischenglieder-Set (72) für ein Stethoskop,
umfassend zwei Zwischenglieder (42; 42a; 42b) jeweils zur Anordnung zwischen einem Ohrbügelende (1) und einer Ohrolive (41) des Stethoskops,
wobei die Zwischenglieder (42; 42a; 42b) jeweils aufweisen
- einen Eingangskanal (EK) für eingehenden Schall und einen Eingangsanschluss (13) des Eingangskanals (EK) zur Verbindung mit dem Ohrbügelende (1), und
- einen Ausgangskanal (AK) für ausgehenden Schall und einen Ausgangsanschluss (14) des Ausgangskanals (AK) zur Verbindung mit der Ohrolive (41),
- eine Mikrofon-Anordnung (9) zur Registrierung des eingehenden Schalls aus dem Eingangskanal (EK),
- eine Verstärker-Anordnung (73) zur elektronischen Verstärkung des eingehenden Schalls, und
- eine Lautsprecher-Anordnung (10) zur Ausgabe des ausgehenden Schalls in den Ausgangskanal (AK),
**dadurch gekennzeichnet,**
**dass** die Zwischenglieder (42; 42a; 42b) jeweils so ausgebildet sind, dass
- der Eingangskanal (EK) oder eine eingangsseitige Schallkammer (15), in die der Eingangskanal (EK) mündet, und der Ausgangskanal (AK) oder eine ausgangsseitige Schallkammer (16), in die der Ausgangskanal (AK) mündet, durch eine feste Zwischenwand (7) des Zwischenglieds (42; 42a; 42b) akustisch voneinander getrennt sind,
- **dass** die Mikrofon-Anordnung (9) axial endseitig am Eingangskanal (EK) und zumindest teilweise innerhalb eines radialen Erstreckungsbereichs (RBE) des Eingangskanals (EK) angeordnet ist,
- und **dass** die Lautsprecher-Anordnung (10) axial endseitig am Ausgangskanal (AK) und zumindest teilweise innerhalb eines radialen Erstreckungsbereichs (RBA) des Ausgangskanals (AK) angeordnet ist.

2. Set (72) nach Anspruch 1, **dadurch gekennzeichnet, dass** die Zwischenglieder (42; 42a; 42b) jeweils so ausgebildet sind, dass
- der Ausgangskanal (AK) quer zu einer axialen Erstreckungsrichtung (ERE) des Eingangskanals (EK) gegenüber dem Eingangskanal (EK) versetzt angeordnet ist.

3. Set (72) nach Anspruch 2, **dadurch gekennzeichnet, dass** der Eingangskanal (EK) und der Ausgangskanal (AK) zumindest näherungsweise parallel zueinander ausgerichtet sind.

4. Set (72) nach Anspruch 2 oder 3, **dadurch gekennzeichnet, dass** die Zwischenglieder (42; 42a; 42b) jeweils so ausgebildet sind, dass
- die Mikrofon-Anordnung (9) und die Lautsprecher-Anordnung (10) bezüglich der axialen Erstreckungsrichtung (ERE) des Eingangskanals (EK) überlappen und/oder die Lautsprecher-Anordnung (10) bezüglich der axialen Erstreckungsrichtung (ERE) des Eingangskanals (EK) näher an einer Eingangsseite des Eingangskanals (EK) liegt als die Mikrofon-Anordnung (9).

5. Set (72) nach einem der vorhergehenden Ansprüche, **dadurch gekennzeichnet, dass** die Mikrofon-Anordnung (9) eine bevorzugte Registrierungsrichtung (BRR) für Schall aufweist, die entlang der axialen Erstreckungsrichtung (ERE) des Eingangskanals (EK) in den Eingangskanal (EK) gerichtet ist,
und dass die Lautsprecher-Anordnung (10) eine bevorzugte Ausgaberichtung (BAR) für Schall aufweist, die entlang der axialen Erstreckungsrichtung (ERA) des Ausgangskanals (AK) in den Ausgangskanal (AK) gerichtet ist.

6. Set (72) nach einem der vorhergehenden Ansprüche, **dadurch gekennzeichnet, dass** die Zwischenglieder (42; 42a; 42b) jeweils so ausgebildet sind, dass der Eingangsanschluss (13) durch einen Eingangsadapter (2) ausgebildet ist, der in einem Gehäuse (4) des Zwischenglieds (42; 42a; 42b) verrastbar ist, insbesondere mit einer Bajonettmechanik (3).

7. Set (72) nach einem der vorhergehenden Ansprüche, **dadurch gekennzeichnet, dass** die Verstärker-Anordnung (73) und ein Energiespeicher (25) für die Verstärker-Anordnung (73) zumindest teilweise, bevorzugt überwiegend, besonders bevorzugt vollständig, in einem Bauraum (5) angeordnet sind, der bezüglich der axialen Erstreckungsrichtung (ERE) des Eingangskanals (EK) mit dem Eingangskanal (EK) überlappt.

8. Set (72) nach einem der vorhergehenden Ansprüche, **dadurch gekennzeichnet, dass** die beiden Zwischenglieder (42; 42a; 42b) mit Zusatz-Mikrofonanordnungen (43) ausgestattet sind, die den Umgebungsschall aufnehmen und phasenverkehrt in die Verstärker-Anordnung (73) einkoppeln.

9. Set (72) nach einem der vorhergehenden Ansprüche, **dadurch gekennzeichnet, dass** die beiden Zwischenglieder (42; 42a; 42b) mit Funkmodulen (24) ausgestattet sind, mittels denen die Zwischenglieder (42; 42a; 42b) eine oder mehrere Einstellungen der Verstärker-Anordnungen (73), insbesondere Lautstärke-Einstellungen und/oder Frequenzgang-Einstellungen, kommunizieren können, und dass zur Vorgabe dieser einen oder mehreren Einstellungen der Verstärker-Anordnungen (73) lediglich an einem der Zwischenglieder (42; 42a; 42b) ein oder mehrere manuelle Bedienelemente (6; 21, 23) vorgesehen sind.

10. Set (72) nach einem der vorhergehenden Ansprüche, **dadurch gekennzeichnet, dass** die Zwischenglieder (42; 42a; 42b) wiederaufladbare Energiespeicher (25) aufweisen, insbesondere wobei die Zwischenglieder (42; 42a; 42b) Ladeelektroniken (26) mit Überladungs- und Tiefentladeschutz aufweisen.

11. Set (72) nach Anspruch 10, **dadurch gekennzeichnet, dass** an einer oder mehreren Außenseiten der Zwischenglieder (42; 42a; 42b) Photovoltaik-Module (30) angeordnet sind, mit denen die wiederaufladbaren Energiespeicher (25) der Zwischenglieder (42; 42a; 42b) aufladbar sind.

12. Set (72) nach einem der vorhergehenden Ansprüche, **dadurch gekennzeichnet, dass** die Eingangsanschlüsse (13) jeweils mit einer Verdrehsicherung oder Verdrehhemmung für die Ohrbügelenden (1) ausgebildet sind.

13. Stethoskop-System (70), umfassend
- ein Stethoskop-Bruststück (37) mit einem oder zwei Bruststück-Ausgängen (37a),
- ein oder zwei Ohrbügel (39) mit je einem Ohrbügel-Eingang (39a) und insgesamt zwei Ohrbügelenden (1),
- eine oder zwei Schlauchleitungen (38) vom Bruststück-Ausgang (37a) oder von den Bruststück-Ausgängen (37a) zum Ohrbügel-Eingang (39a) oder zu den Ohrbügel-Eingängen (39a),
- ein Zwischenglieder-Set (72) nach einem der vorhergehenden Ansprüche, wobei jeweils ein Ohrbügelende (1) an einem Eingangsanschluss (13) eines Zwischenglieds (42; 42a; 42b) angeschlossen ist,
- und zwei Ohroliven (41), die jeweils an einem Ausgangsanschluss (14) eines Zwischenglieds (42; 42a; 42b) angeordnet sind.

14. Stethoskop-System (70) nach Anspruch 13 in Verbindung mit einem Zwischenglieder-Set (42; 42a; 42b) nach Anspruch 2, **dadurch gekennzeichnet, dass** die Zwischenglieder (42; 42a; 42b) so an dem Ohrbügel (39) oder den Ohrbügeln (39) angeordnet sind, dass die Eingangskanäle (EK) dem Ohrbügel-Eingang (39a) oder den Ohrbügel-Eingängen (39a) abgewandt sind, und die Ausgangskanäle (AK) dem Ohrbügel-Eingang (39a) oder den Ohrbügel-Eingängen (39a) zugewandt sind.

15. Stethoskop-System (70) nach Anspruch 13 oder 14 in Verbindung mit einem Set (72) nach Anspruch 10, weiterhin umfassend eine Ladestation (74) mit zwei virtuellen Ohren (75), an denen die an dem oder den Ohrbügeln (39) montierten Zwischenglieder (42; 42a; 42b) mit den Ohroliven (41) einhängbar sind, wobei im eingehängten Zustand die Zwischenglieder (42; 42a; 42b) so angeordnet sind, dass die Ladestation (74) die wiederaufladbaren Energiespeicher (25) der Zwischenglieder (42; 42a; 42b) aufladen kann.

16. Stethoskop-System (70) nach Anspruch 15, **dadurch gekennzeichnet, dass** der Abstand zwischen den beiden virtuellen Ohren (75) variabel einstellbar ist.

17. Stethoskop-System (70) nach Anspruch 15 oder 16, **dadurch gekennzeichnet, dass** die Ladestation (74) ausgebildet ist mit
- einem auf die Ladestation (74) aufsteckbaren Photovoltaik-Modul (44) zur Energieversorgung, und
- einem Kabel (77) zur Verbindung des Photovoltaik-Moduls (44) mit der Ladestation (74) zumindest im abgesteckten Zustand des Photovoltaik-Moduls (44).

18. Stethoskop-System (70) nach Anspruch 17, **dadurch**
**gekennzeichnet, dass** die Ladestation (74) einen wiederaufladbaren Puffer-Energiespeicher (55) umfasst.

19. Stethoskop-System (70) nach einem der Ansprüche 15 bis 18, **dadurch gekennzeichnet, dass** die Ladestation (74) mit einer Diebstahlsicherung (78) ausgebildet ist, umfassend zwei verfahrbare Platten (63), die bei an den virtuellen Ohren (75) angeordneten Zwischengliedern (42; 42a; 42b) von außen auf Anschlag an die Zwischenglieder (42; 42a; 42b) und/oder an den oder die Ohrbügel (39), an dem oder denen die Zwischenglieder (42; 42a; 42b) montiert sind, heranfahrbar und am Anschlag verrastbar sind, wobei die Verrastung der Platten (33) mit einem Schloss aufhebbar ist.

## Claims

1. An intermediate link set (72) for a stethoscope,
comprising two intermediate links (42; 42a; 42b) each for arrangement between an ear-hook end (1) and an earpiece (41) of the stethoscope,
the intermediate links (42; 42a; 42b) each having
- an input channel (EK) for incoming sound and an input connector (13) of the input channel (EK) for connection to the ear-hook end (1), and
- an output channel (AK) for outgoing sound and an output connector (14) of the output channel (AK) for connection to the earpiece (41),
- a microphone arrangement (9) for recording the incoming sound from the input channel (EK),
- an amplifier arrangement (73) for electronically amplifying the incoming sound,and
- a loudspeaker arrangement (10) for outputting the outgoing sound into the output channel (AK),
**characterized in that**
the intermediate links (42; 42a; 42b) are each designed such that
- the input channel (EK) or an input-side sound chamber (15), into which the input channel (EK) opens, and the output channel (AK) or an output-side sound chamber (16), into which the output channel (AK) opens, are acoustically separated from one another by a fixed intermediate wall (7) of the intermediate link (42; 42a; 42b),
- the microphone arrangement (9) is arranged axially at the end of the input channel (EK) and at least partially within a radial extension region (RBE) of the input channel (EK),
- and the loudspeaker arrangement (10) is arranged axially at the end of the output channel (AK) and at least partially within a radial extension region (RBA) of the output channel (AK).

2. The set (72) according to claim 1, **characterized in that** the intermediate links (42; 42a; 42b) are each designed such that
- the output channel (AK) is arranged so as to be offset relative to the input channel (EK) transversely to an axial extension direction (ERE) of the input channel (EK).

3. The set (72) according to claim 2, **characterized in that** the input channel (EK) and the output channel (AK) are oriented at least approximately parallel to one another.

4. The set (72) according to claim 2 or claim 3, **characterized in that** the intermediate links (42; 42a; 42b) are each designed such that
- the microphone arrangement (9) and the loudspeaker arrangement (10) overlap with respect to the axial extension direction (ERE) of the input channel (EK) and/or the loudspeaker arrangement (10) is closer to an input side of the input channel (EK) with respect to the axial extension direction (ERE) of the input channel (EK) than the microphone arrangement (9).

5. The set (72) according to any of the preceding claims,
**characterized in that** the microphone arrangement (9) has a preferred recording direction (BRR) for sound, which direction is directed along the axial extension direction (ERE) of the input channel (EK) into the input channel (EK),
and **in that** the loudspeaker arrangement (10) has a preferred output direction (BAR) for sound, which direction is directed along the axial extension direction (ERA) of the output channel (AK) into the output channel (AK).

6. The set (72) according to any of the preceding claims, **characterized in that** the intermediate links (42; 42a; 42b) are each designed such that the input connector (13) is formed by an input adapter (2) which can be latched in a housing (4) of the intermediate link (42; 42a; 42b), in particular by means of a bayonet mechanism (3).

7. The set (72) according to any of the preceding claims, **characterized in that** the amplifier arrangement (73) and an energy store (25) for the amplifier arrangement (73) are arranged at least partially, preferably predominantly, particularly preferably completely, in an installation space (5) which overlaps with the input channel (EK) with respect to the axial extension direction (ERE) of the input channel (EK).

8. The set (72) according to any of the preceding claims,
**characterized in that** the two intermediate links (42; 42a; 42b) are provided with additional microphone arrangements (43) which receive the ambient sound and couple said sound into the amplifier assembly (73) in a phase-inverted manner.

9. The set (72) according to any of the preceding claims, **characterized in that** the two intermediate links (42; 42a; 42b) are provided with radio modules (24) by means of which the intermediate links (42; 42a; 42b) can communicate one or more settings of the amplifier arrangements (73), in particular volume settings and/or frequency response settings, and **in that,** for specifying these one or more settings of the amplifier arrangements (73), one or more manual operating elements (6; 21, 23) are provided only on one of the intermediate links (42; 42a; 42b).

10. The set (72) according to any of the preceding claims,
**characterized in that** the intermediate links (42; 42a; 42b) have rechargeable energy stores (25), in particular the intermediate links (42; 42a; 42b) having charging electronics (26) that have overload and deep-discharge protection.

11. The set (72) according to claim 10, **characterized in that** photovoltaic modules (30) are arranged on one or more outer sides of the intermediate links (42; 42a; 42b), by means of which modules the rechargeable energy stores (25) of the intermediate links (42; 42a; 42b) can be charged.

12. The set (72) according to any of the preceding claims,
**characterized in that** the input connectors (13) are each designed to have an anti-rotation element or anti-rotation block for the ear-hook ends (1).

13. A stethoscope system (70), comprising
- a stethoscope chest piece (37) having one or two chest piece outputs (37a),
- one or two ear hooks (39) each having an ear-hook input (39a) and having a total of two ear-hook ends (1),
- one or two tube lines (38) from the chest piece output (37a) or from the chest piece outputs (37a) to the ear-hook input (39a) or to the ear-hook inputs (39a),
- an intermediate link set (72) according to any of the preceding claims, in each case an ear-hook end (1) being connected to an input connector (13) of an intermediate link (42; 42a; 42b),
- and two earpieces (41) which are each arranged on an output connector (14) of an intermediate link (42; 42a; 42b).

14. The stethoscope system (70) according to claim 13 in conjunction with an intermediate link set (42; 42a; 42b) according to claim 2, **characterized in that** the intermediate links (42; 42a; 42b) are arranged on the ear hook (39) or the ear hooks (39) such that the input channels (EK) face away from the ear-hook input (39a) or the ear-hook inputs (39a), and the output channels (AK) face the ear-hook input (39a) or the ear-hook inputs (39a).

15. The stethoscope system (70) according to claim 13 or claim 14 in conjunction with a set (72) according to claim 10, further comprising a charging station (74) having two virtual ears (75) on which the intermediate links (42; 42a; 42b) mounted on the ear hook(s) (39) can be suspended by means of the earpieces (41), wherein, in the suspended state, the intermediate links (42; 42a; 42b) are arranged such that the charging station (74) can charge the rechargeable energy stores (25) of the intermediate links (42; 42a; 42b).

16. The stethoscope system (70) according to claim 15, **characterized in that** the distance between the two virtual ears (75) is variably adjustable.

17. The stethoscope system (70) according to claim 15 or claim 16,
**characterized in that** the charging station (74) is designed to have
- a photovoltaic module (44) attachable to the charging station (74) for power supply, and
- a cable (77) for connecting the photovoltaic module (44) to the charging station (74) at least when the photovoltaic module (44) is detached.

18. The stethoscope system (70) according to claim 17, **characterized in that** the charging station (74) comprises a rechargeable buffer energy store (55).

19. The stethoscope system (70) according to any of claims 15 to 18,
**characterized in that** the charging station (74) is designed to have an anti-theft device (78), comprising two movable plates (63) which, when intermediate links (42; 42a; 42b) are arranged on the virtual ears (75), can be moved from the outside to stop against the intermediate links (42; 42a; 42b) and/or against the ear hook(s) (39) on which the intermediate links (42; 42a; 42b) are mounted, and can be latched against the stop, it being possible to release the latching of the plates (33) using a lock.

## Revendications

1. Ensemble (72) de pièces intermédiaires, destiné à un stéthoscope et incluant deux pièces intermédiaires (42 ; 42a ; 42b) respectivement conçues pour être interposées entre une extrémité (1) de tube auriculaire cintré et un embout auriculaire (41) du stéthoscope,
lesquelles pièces intermédiaires (42 ; 42a ; 42b) comprennent, à chaque fois,
- un canal d'entrée (EK) dévolu à un son entrant et un raccord d'entrée (13) dudit canal d'entrée (EK), affecté à la liaison avec l'extrémité (1) de tube auriculaire cintré, et
- un canal de sortie (AK) dévolu à un son sortant et un raccord de sortie (14) dudit canal de sortie (AK), affecté à la liaison avec l'embout auriculaire (41),
- un dispositif microphonique (9), dédié à l'enregistrement du son entrant émanant du canal d'entrée (EK),
- un dispositif amplificateur (73) dédié à l'amplification électronique dudit son entrant, et
- un dispositif haut-parleur (10), conçu pour délivrer le son sortant dans le canal de sortie (AK),
**caractérisé par le fait**
**que** les pièces intermédiaires (42 ; 42a ; 42b) sont respectivement réalisées de façon telle que
- le canal d'entrée (EK), ou une chambre acoustique (15) située côté entrée et dans laquelle ledit canal d'entrée (EK) débouche, et le canal de sortie (AK), ou une chambre acoustique (16) située côté sortie et dans laquelle ledit canal de sortie (AK) débouche, sont séparés acoustiquement les uns des autres par une cloison intermédiaire fixe (7) de la pièce intermédiaire (42 ; 42a ; 42b) ;
- par le fait que le dispositif microphonique (9) est implanté axialement à l'extrémité du canal d'entrée (EK) et, au moins en partie, à l'intérieur d'une zone d'étendue radiale (RBE) dudit canal d'entrée (EK) ;
- et par le fait que le dispositif haut-parleur (10) est implanté axialement à l'extrémité du canal de sortie (AK) et, au moins en partie, à l'intérieur d'une zone d'étendue radiale (RBA) dudit canal de sortie (AK).

2. Ensemble (72) selon la revendication 1, **caractérisé par le fait que** les pièces intermédiaires (42 ; 42a ; 42b) sont respectivement réalisées de façon telle que
- le canal de sortie (AK) soit agencé, relativement au canal d'entrée (EK), avec décalage transversal par rapport à une direction d'étendue axiale (ERE) dudit canal d'entrée (EK).

3. Ensemble (72) selon la revendication 2, **caractérisé par le fait que** le canal d'entrée (EK) et le canal de sortie (AK) sont orientés parallèlement l'un à l'autre, au moins approximativement.

4. Ensemble (72) selon la revendication 2 ou 3, **caractérisé par le fait que** les pièces intermédiaires (42 ; 42a ; 42b) sont respectivement réalisées de façon telle que
- le dispositif microphonique (9) et le dispositif haut-parleur (10) se chevauchent par rapport à la direction (ERE) de l'étendue axiale du canal d'entrée (EK) ; et/ou que, par rapport à ladite direction (ERE) de l'étendue axiale du canal d'entrée (EK), ledit dispositif haut-parleur (10) soit plus rapproché d'un côté entrée dudit canal d'entrée (EK) que ledit dispositif microphonique (9).

5. Ensemble (72) selon l'une des revendications précédentes, **caractérisé par le fait que** le dispositif microphonique (9) présente une direction préférentielle (BRR) d'enregistrement du son qui pointe, vers le canal d'entrée (EK), le long de la direction (ERE) de l'étendue axiale dudit canal d'entrée (EK) ;
et **par le fait que** le dispositif haut-parleur (10) présente une direction préférentielle (BAR) de délivrance du son qui pointe, vers le canal de sortie (AK), le long de la direction (ERA) de l'étendue axiale dudit canal de sortie (AK).

6. Ensemble (72) selon l'une des revendications précédentes, **caractérisé par le fait que** les pièces intermédiaires (42 ; 42a ; 42b) sont respectivement réalisées de façon telle que le raccord d'entrée (13) soit matérialisé par un adaptateur d'entrée (2) pouvant être encliqueté dans un boîtier (4) de la pièce intermédiaire (42 ; 42a ; 42b), notamment à l'aide d'un mécanisme à baïonnette (3).

7. Ensemble (72) selon l'une des revendications précédentes, **caractérisé par le fait que** le dispositif amplificateur (73) et un accumulateur d'énergie (25) destiné audit dispositif amplificateur (73) sont logés au moins en partie, de préférence en majeure partie et, avec préférence particulière, en totalité dans un espace structurel (5) se chevauchant avec le canal d'entrée (EK) par rapport à la direction (ERE) de l'étendue axiale dudit canal d'entrée (EK).

8. Ensemble (72) selon l'une des revendications précédentes, **caractérisé par le fait que** les deux pièces intermédiaires (42 ; 42a ; 42b) sont équipées de dispositifs microphoniques (43) additionnels, qui captent le son environnant et l'introduisent dans le dispositif amplificateur (73) avec inversion de phase.

9. Ensemble (72) selon l'une des revendications précédentes, **caractérisé par le fait que** les deux pièces intermédiaires (42 ; 42a ; 42b) sont équipées de modules radio (24) au moyen desquels lesdites pièces intermédiaires (42 ; 42a ; 42b) peuvent communiquer un ou plusieurs réglage(s) des dispositifs amplificateurs (73), en particulier des réglages d'intensité sonore et/ou des réglages de réponse en fréquence ; et **par le fait qu'**un ou plusieurs élément(s) (6 ; 21 ; 23) de pilotage manuel est (sont) prévu(s), uniquement sur l'une desdites pièces intermédiaires (42 ; 42a ; 42b), pour préétablir ces réglages uniques ou multiples desdits dispositifs amplificateurs (73).

10. Ensemble (72) selon l'une des revendications précédentes, **caractérisé par le fait que** les pièces intermédiaires (42 ; 42a ; 42b) sont pourvues d'accumulateurs d'énergie (25) rechargeables, lesdites pièces intermédiaires (42 ; 42a ; 42b) présentant notamment des électroniques de charge (26) dotées d'une protection contre les surcharges et les décharges intégrales.

11. Ensemble (72) selon la revendication 10, **caractérisé par le fait que** des modules photovoltaïques (30), à l'aide desquels les accumulateurs d'énergie rechargeables (25) des pièces intermédiaires (42 ; 42a ; 42b) peuvent être chargés, sont implantés sur une ou plusieurs face(s) extérieure(s) desdites pièces intermédiaires (42 ; 42a ; 42b).

12. Ensemble (72) selon l'une des revendications précédentes, **caractérisé par le fait que** les raccords d'entrée (13) sont munis, à chaque fois, d'une sécurité antirotation ou d'une entrave à la rotation équipant les extrémités (1) des tubes auriculaires cintrés.

13. Système stéthoscopique (70) comprenant
- un pavillon stéthoscopique (37) doté d'une ou de deux sortie(s) (37a),
- un ou deux tube(s) auriculaire(s) cintré(s) (39), pourvu(s) d'une entrée (39a) respective et de deux extrémités (1) au total,
- un ou deux conduit(s) souple(s) (38) qui partent de la sortie (37a) du pavillon ou des sorties (37a) dudit pavillon, et gagnent l'entrée (39a) d'un tube auriculaire cintré ou les entrées (39a) des tubes auriculaires cintrés,
- un ensemble (72) de pièces intermédiaires conforme à l'une des revendications précédentes, une extrémité (1) de tube auriculaire cintré étant respectivement reliée à un raccord d'entrée (13) d'une pièce intermédiaire (42 ; 42a ; 42b),
- et deux embouts auriculaires (41) disposés, à chaque fois, au niveau d'un raccord de sortie (14) d'une pièce intermédiaire (42 ; 42a ; 42b).

14. Système stéthoscopique (70) selon la revendication 13, combiné à un ensemble de pièces intermédiaires (42 ; 42a ; 42b) conforme à la revendication 2, **caractérisé par le fait que** les pièces intermédiaires (42 ; 42a ; 42b) occupent, sur le tube auriculaire cintré (39) ou sur les tubes auriculaires cintrés (39), des emplacements tels que les canaux d'entrée (EK) pointent à l'opposé de l'entrée (39a) dudit tube auriculaire cintré ou des entrées (39a) desdits tubes auriculaires cintrés, et que les canaux de sortie (AK) pointent vers ladite entrée (39a) du tube auriculaire cintré ou vers lesdites entrées (39a) des tubes auriculaires cintrés.

15. Système stéthoscopique (70) selon la revendication 13 ou 14, combiné à un ensemble (72) conforme à la revendication 10, incluant par ailleurs un poste de charge (74) muni de deux oreilles virtuelles (75) auxquelles les pièces intermédiaires (42 ; 42a ; 42b), montées sur le ou les tube(s) auriculaire(s) cintré(s) (39), peuvent être accrochées par les embouts auriculaires (41), sachant que lesdites pièces intermédiaires (42 ; 42a ; 42b) occupent, à l'état accroché, des emplacements tels que ledit poste de charge (74) puisse charger les accumulateurs d'énergie rechargeables (25) desdites pièces intermédiaires (42 ; 42a ; 42b).

16. Système stéthoscopique (70) selon la revendication 15, **caractérisé par le fait que** la distance séparant les deux oreilles virtuelles (75) peut être réglée de manière variable.

17. Système stéthoscopique (70) selon la revendication 15 ou 16, **caractérisé par le fait que** le poste de charge (74) est doté
- d'un module photovoltaïque (44) pouvant être enfiché sur ledit poste de charge (74), en vue de l'alimentation en énergie, et
- d'un câble (77) dévolu à la connexion dudit module photovoltaïque (44) avec ledit poste de charge (74), au moins à l'état désenfiché dudit module photovoltaïque (44).

18. Système stéthoscopique (70) selon la revendication 17, **caractérisé par le fait que** le poste de charge (74) inclut un accumulateur d'énergie tampon (55) rechargeable.

19. Système stéthoscopique (70) selon l'une des revendications 15 à 18, **caractérisé par le fait que** le poste de charge (74) est équipé d'une sécurité antivol (78) comprenant deux platines mobiles (63) qui, lorsque les pièces intermédiaires (42 ; 42a ; 42b) sont placées sur les oreilles virtuelles (75), peuvent être présentées de l'extérieur, avec venue en butée, auxdites pièces intermédiaires (42 ; 42a ; 42b) et/ou au(x) tube(s) auriculaire(s) cintré(s) (39) sur le(s)quel(s) lesdites pièces intermédiaires (42 ; 42a ; 42b) sont montées, et peuvent être encliquetées sur la butée, l'encliquetage desdites platines (33) pouvant être neutralisé à l'aide d'une clé.
